# EUROPEAN PATENT APPLICATION

(11) **EP 4 134 204 A1**
(43) Date of publication of application: **15.02.2023**
(21) Application number: 21784024.8
(22) Date of filing: 08.04.2021
(51) Int. Cl.: B25J 3/00, B25J 5/00, A61B 34/35, A61B 34/37, A61B 90/50, A61B 90/70, A61G 12/00

(54) **ROBOT SYSTEM AND METHOD FOR OPERATING SAME**

(30) Priority: 10.04.2020 JP 2020071340; 29.05.2020 JP 2020094589
(71) Applicant: KAWASAKI JUKOGYO KABUSHIKI KAISHA, Kobe-shi, Hyogo 650-8670 (JP)
(72) Inventor: HASHIMOTO, Yasuhiko, Hyogo 650-8670 (JP); KAMEYAMA, Atsushi, Hyogo 650-8670 (JP); KAMON, Masayuki, Hyogo 650-8670 (JP)
(74) Representative: Müller-Boré & Partner Patentanwälte PartG mbB
(86) International application number: PCT/JP2021/014954
(87) International publication number: WO 2021/206153

(57) **Abstract**

In a robot system (100), a controller (110) is confi gured to cause a robot (101) to be self-propelled to approach a patient, and then operate an arm ( 13) and/or a hand (18) based on operation command informat ion of the arm (13) and/or the hand (18), which has been i nput from an operator (102).

## Description

### Technical Field

The present invention relates to a robot system and a method for operating the same.

### Background Art

A self-propelled tray transfer robot that manages the presence or absence of a patient and is self-propelled to supply a blood collection tube containment tray of a patient, which is prepared in a blood collection tube preparation room, to a blood collection table is known (for example, see PTL 1).

In the self-propelled tray transfer robot disclosed in PTL 1, a non-contact medium such as an RF-ID is used as the test acceptance slip, and a reader for the non-contact medium is installed at the entrance gate of the blood collection room to check the entry and exit of the patient.

When the patient is absent, the self-propelled tray transfer robot receives the tray automatically prepared by a blood collection tube preparation device and stocks the received tray in a predetermined stock section. When the patient is present in a blood collection room, medical practice is assisted in a manner that a self-propelled tray transfer robot is self-propelled to supply a tray to the requested blood collection table.

### Citation List

### Patent Literature

[PTL 1] Japanese Unexamined Patent Publication No. 2007-130282

### Summary of Invention

In recent years, infectious diseases caused by coronaviruses such as SARS, MERS, and COVID-19 have spread. Infectious diseases caused by viruses other than coronavirus or various bacteria are also well known.

For patients suspected of having these infectious diseases, a specimen such as mucous membrane is collected, and a test such as a PCR test is performed to diagnose the presence or absence of infection. There is a concern that a medical staff that collects the specimen is infected with viruses.

In addition, there is a concern that a medical staff who comes into contact with a patient infected with a virus or the like moves in the hospital during a period from when the test is performed until the infection is confirmed, so patients with other diseases in the hospital are infected with the virus or the like.

Therefore, the inventor of the present application has found that it is possible to sufficiently reduce the infection of a medical staff and the like in a hospital by causing a robot by a remote operation to perform a test and an examination on a patient suspected of being infected with a virus or the like, and has made the present invention.

An object of the present invention is to provide a robot system and a method for operating the robot system capable of sufficiently reducing infection of a medical staff in a hospital.

In order to solve the above-described conventional problems, according to the present invention, a robot system includes a robot including an arm with a hand that holds a medical test instrument and/or a medical examination instrument, an operator configured to operate the robot, and a controller. A first space in which the robot is disposed is isolated from a second space in which the operator is disposed, and the controller is configured to (A) cause the robot to be self-propelled to approach the patient, and (B) operate the arm and/or the hand based on operation command information of the arm and/or the hand, which has been input from the operator, after (A) is performed.

As a result, since a medical staff operates the robot in a space in which the medical staff is isolated from a patient, it is possible to suppress a contact of the medical staff with a patient suspected of being infected with a virus or the like. Therefore, it is possible to sufficiently suppress the infection to the medical staff with a virus or the like.

Further, since the robot moves to the vicinity of the patient in each hospitalization room or the like, the medical staff does not need to move in the hospital. Therefore, it is possible to sufficiently suppress the infection to the medical staff.

Furthermore, by separately using a robot for a patient suspected of having an infectious disease and a robot for a patient with another disease, it is possible to sufficiently suppress the infection to a patient with another disease.

Further, according to the present invention, a robot system includes a robot including an arm with a hand that holds a medical test instrument and/or a medical examination instrument, an operator configured to operate the robot, and a controller. A first space in which the robot is disposed is isolated from a second space in which the operator is disposed, and a first imager is disposed at the hand. The controller is configured to (α) operate the arm and/or the hand based on operation command information of the arm and/or the hand, which has been input from the operator.

As a result, since a medical staff operates the robot in a space in which the medical staff is isolated from a patient, it is possible to suppress a contact of the medical staff with a patient suspected of being infected with a virus or the like. Therefore, it is possible to sufficiently suppress the infection to the medical staff with a virus or the like.

Further, according to the present invention, there is provided a method for operating a robot system that includes a robot including an arm with a hand that holds a medical test instrument and/or a medical examination instrument and an operator configured to operate the robot. A first space in which the robot is disposed is isolated from a second space in which the operator is disposed. The method includes (A) causing the robot to be self-propelled to approach the patient, and (B) operating the arm and/or the hand based on operation command information of the arm and/or the hand, which has been input from the operator, after (A) is performed.

As a result, since a medical staff operates the robot in a space in which the medical staff is isolated from a patient, it is possible to suppress a contact of the medical staff with a patient suspected of being infected with a virus or the like. Therefore, it is possible to sufficiently suppress the infection to the medical staff with a virus or the like.

Further, since the robot moves to the vicinity of the patient in each hospitalization room or the like, the medical staff does not need to move in the hospital. Therefore, it is possible to sufficiently suppress the infection to the medical staff.

Furthermore, by separately using a robot for a patient suspected of having an infectious disease and a robot for a patient with another disease, it is possible to sufficiently suppress the infection to a patient with another disease.

Further, according to the present invention, there is provided a method for operating a robot system that includes a robot including an arm with a hand that holds a medical test instrument and/or a medical examination instrument, and an operator configured to operate the robot. A first space in which the robot is disposed is isolated from a second space in which the operator is disposed, and a first imager is disposed at the hand. The method includes (α) operating the arm and/or the hand based on operation command information of the arm and/or the hand, which has been input from the operator.

As a result, since a medical staff operates the robot in a space in which the medical staff is isolated from a patient, it is possible to suppress a contact of the medical staff with a patient suspected of being infected with a virus or the like. Therefore, it is possible to sufficiently suppress the infection to the medical staff with a virus or the like.

The above object, other objects, features, and advantages of the present invention will become apparent from the following detailed description of preferred embodiments with reference to the accompanying drawings.

According to the robot system and the method for operating the same of the present invention, it is possible to sufficiently reduce the infection of a medical staff and the like in a hospital.

### Brief Description of Drawings

FIG. 1 is a schematic diagram illustrating a schematic configuration of a robot system according to Embodiment 1.
FIG. 2 is a schematic diagram illustrating the schematic configuration of the robot system according to Embodiment 1.
FIG. 3 is a schematic diagram illustrating a schematic configuration of a robot in the robot system illustrated in FIGS. 1 and 2.
FIG. 4 is a schematic diagram illustrating a schematic configuration of a hand of the robot in the robot system according to Embodiment 1.
FIG. 5 is a flowchart illustrating an example of an operation of the robot system according to Embodiment 1.
FIG. 6 is a schematic diagram illustrating an example of an operation of the robot in the robot system according to Embodiment 1.
FIG. 7 is a schematic diagram illustrating an example of image information and/or video information displayed on a first display illustrated in FIG. 1.
FIG. 8 is a schematic diagram illustrating another example of the image information and/or the video information displayed on the first display illustrated in FIG. 1.
FIG. 9 is a schematic diagram illustrating yet another example of the image information and/or the video information displayed on the first display illustrated in FIG. 1.
FIG. 10 is a schematic diagram illustrating still yet another example of the image information and/or the video information displayed on the first display illustrated in FIG. 1.
FIG. 11 is a schematic diagram illustrating a schematic configuration of a robot system according to Modification Example 1 in Embodiment 1.
FIG. 12 is a schematic diagram illustrating a schematic configuration of a robot system according to Embodiment 2.
FIG. 13 is a schematic diagram illustrating a schematic configuration of a robot system according to Embodiment 3.
FIG. 14 is a schematic diagram illustrating a schematic configuration of a robot system according to Embodiment 4.
FIG. 15 is a flowchart illustrating an example of an operation of the robot system according to Embodiment 4.
FIG. 16 is a schematic diagram illustrating a schematic configuration of a robot system according to Modification Example 1 in Embodiment 4.
FIG. 17 is a schematic diagram illustrating a schematic configuration of a robot system according to Embodiment 5.
FIG. 18 is a flowchart illustrating an example of an operation of the robot system according to Embodiment 5.
FIG. 19 is a schematic diagram illustrating a schematic configuration of a robot system according to Embodiment 6.
FIG. 20 is a schematic diagram illustrating the schematic configuration of the robot system according to Embodiment 6.
FIG. 21 is a schematic diagram illustrating a schematic configuration of a hand of a robot illustrated in FIG. 19.
FIG. 22 is a flowchart illustrating an example of an operation of the robot system according to Embodiment 6.
FIG. 23 is a schematic diagram illustrating a schematic configuration of a robot system according to Modification Example 1 in Embodiment 6.
FIG. 24 is a schematic diagram illustrating a schematic configuration of a robot system according to Embodiment 7.
FIG. 25 is a schematic diagram illustrating a schematic configuration of a robot system according to Embodiment 8.
FIG. 26 is a flowchart illustrating an example of an operation of the robot system according to Embodiment 8. Description of Embodiments

Hereinafter, embodiments of the present invention will be described with reference to the drawings. In all drawings, the same or corresponding portions are designated by the same reference signs, and duplicate description will be omitted. Further, in all the drawings, the components for describing the present invention are excerpted and illustrated, and the illustrations of other components may be omitted. Furthermore, the present invention is not limited to the following embodiments.

### (Embodiment 1)

A robot system according to Embodiment 1 includes a robot, an operator, and a controller. The robot includes an arm with a hand that holds a medical test instrument and/or a medical examination instrument. The operator is configured to operate the robot. A first space in which the robot is disposed is isolated from a second space in which the operator is disposed. The controller is configured to (A) cause the robot to be self-propelled to approach a patient and (B) operate the arm based on operation command information of the arm and/or the hand, which has been input from the operator, after (A) is performed.

Further, the robot system according to Embodiment 1 may further include a first imager and a first display configured to display image information and/or video information obtained by picking-up of the first imager. The controller may be configured to perform (B) in a state where the first display displays the image information and/or the video information obtained by picking-up of the first imager.

Further, in the robot system according to Embodiment 1, the robot and the operator may be configured by a master-slave method.

Further, in the robot system according to Embodiment 1, a pair of laser light indicators may be disposed at the hand so that rays of light with which irradiation is performed from the laser light indicators intersect with each other.

Further, in the robot system according to Embodiment 1, the first imager may be disposed at the robot, or may be disposed at the hand of the robot.

Further, in the robot system according to Embodiment 1, the first display may be configured to display a virtual model showing position information of the medical test instrument and/or the medical examination instrument.

Furthermore, in the robot system according to Embodiment 1, an operation switch configured to perform an instruction to release holding of the medical test instrument and/or the medical examination instrument may be disposed at the operator.

In a method for operating a robot system according to Embodiment 1, the robot system includes a robot including an arm with a hand that holds a medical test instrument and/or a medical examination instrument, and an operator configured to operate the robot. A first space in which the robot is disposed is isolated from a second space in which the operator is disposed. The method includes (A) automatically moving the robot to the vicinity of a patient based on position information of the patient, which has been input from the operator, and (B) operating the arm and/or the hand based on operation command information of the arm and/or the hand, which has been input from the operator, after (A) is performed.

Further, in the method for operating a robot system according to Embodiment 1, the robot system may further include a first imager and a first display configured to display image information and/or video information obtained by picking-up of the first imager. (B) may be performed in a state where the image information and/or the video information obtained by picking-up of the first imager is displayed on the first display.

Further, in the method for operating a robot system according to Embodiment 1, the robot and the operator may be configured by a master-slave method.

Further, in the method for operating a robot system according to Embodiment 1, a pair of laser light indicators may be disposed at the hand so that the rays of light with which irradiation is performed from the laser light indicators intersect with each other.

Further, in the method for operating a robot system according to Embodiment 1, the first imager may be disposed at the robot or at the hand of the robot.

Further, in the method for operating a robot system according to Embodiment 1, the first display may be configured to display a virtual model showing position information of the medical test instrument and/or the medical examination instrument.

Further, in the method for operating a robot system according to Embodiment 1, the first display may be configured to display a virtual model of a medical practice target site of a patient.

Furthermore, in the method for operating a robot system according to Embodiment 1, an operation switch configured to perform an instruction to release holding of the medical test instrument and/or the medical examination instrument may be disposed at the operator.

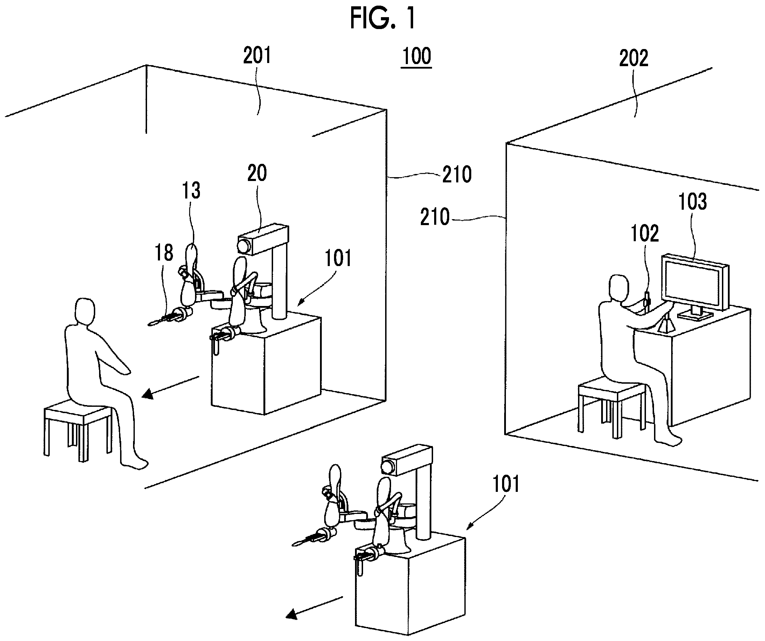

An example of the robot system according to Embodiment 1 will be described below with reference to FIGS. 1 to 10.

### [Configuration of Robot System]

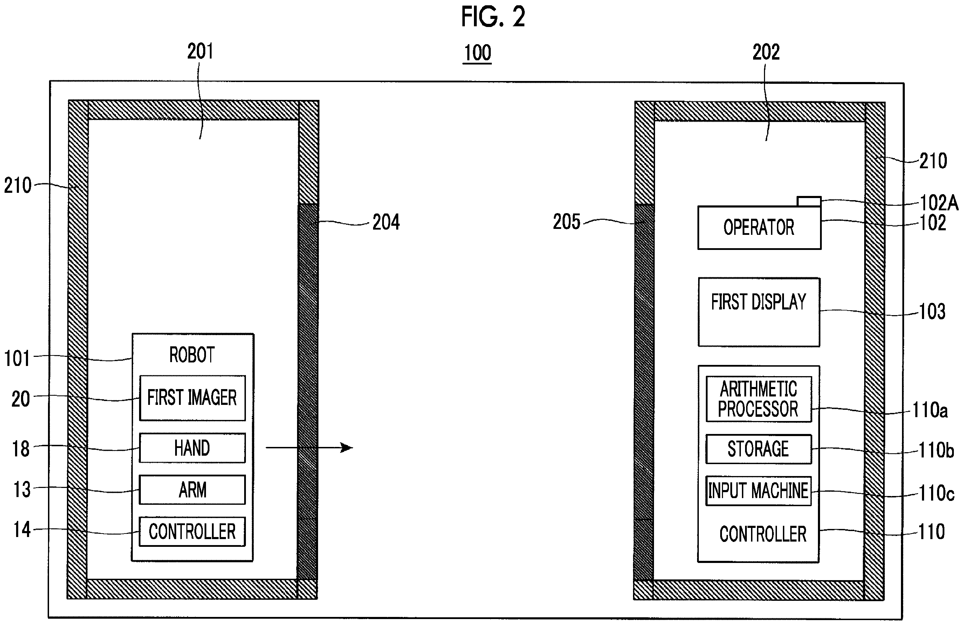

FIGS. 1 and 2 are schematic diagrams illustrating a schematic configuration of a robot system according to Embodiment 1.

As illustrated in FIG. 1, a robot system 100 according to Embodiment 1 includes a robot 101, an operator 102, a first display 103, and a controller 110. The robot 101 is disposed in a first space 201. The operator 102, the first display 103, and the controller 110 are disposed in a second space 202.

The first space 201 and the second space 202 are spaces separated from each other. The first space 201 and the second space 202 are isolated by a partition wall member 210.

An image pickup device (first imager) for picking up an image of a profile of a patient may be disposed in the first space 201. The image pickup device may be installed at the partition wall member 210 forming the first space 201 or may be gripped by a robot different from the robot 101.

In addition, an instrument for performing a medical test by the robot 101, an experimental device, various test reagents, or the like may be disposed in a room (examination room/test room) constituting the first space 201. Examples of the instrument include an autopipette, a tip used for the autopipette, a microtube, a centrifuge tube, and a centrifuge settling tube. Examples of the experimental device include a centrifuge and a PCR device.

An anterior chamber may be provided in a room (operation room) constituting the second space 202. Further, a fan filter unit configured to make the anterior chamber have negative pressure and make the second space 202 (internal space of the operation room) have positive pressure may be installed in the anterior chamber. A known fan filter unit can be used as the fan filter unit.

Further, the partition wall member 210 may include a shutter (door) 204 that permits/prohibits movement into the first space 201, and may further include a shutter (door) 205 that permits/prohibits movement into the second space 202.

Furthermore, the partition wall member 210 may be configured by forming a portion of the partition wall member 210 with a transparent member such as a glass plate so that an operator (medical staff) or the like can view the inside of the first space 201 from the outside.

The operator 102 is configured to operate the robot 101. As the operator 102, for example, a known operator such as a joystick, a keyboard, a numeric keypad, or a teaching pendant can be used.

Further, a device that transmits force sense information detected by a force sense sensor (described later) provided at a hand 18 of the robot 101 and audio information to an operator may be disposed at the operator 102. Examples of such a device include a vibration motor, a speaker, a mechanism for expanding and contracting a housing constituting a grip portion, and the like.

The operator 102 may be configured to be portable and carried by the operator (medical staff). In addition, the robot 101 and the operator 102 may have a master-slave method.

Further, the operator 102 may be provided with a release button 102A for releasing a medical test instrument or a medical examination instrument held by the hand 18 in an emergency (for example, when the robot 101 performs an abnormal operation). When the release button 102A is pressed by the operator, the controller 110 may operate the robot 101 to separate the hand 18 from the patient.

The first display 103 is configured to display image information and/or video information obtained by picking-up of the first imager 20 described later. For example, a stationary type display that is used by being stationary on a desk, floor, or the like may be configured as the first display 103. In addition, the first display 103 may include a head-mounted display or glasses worn and used by the operator.

The robot 101 is configured to be able to be self-propelled to the vicinity of the patient based on the position information of the patient input from the operator 102 and/or position information in a hospital (for example, position information of a hospital room and an examination room).

Further, the robot 101 is configured to operate the arm and/or the hand based on operation information of the arm and/or the hand, which is input from the operator 102. At this time, the robot 101 may be configured so that the robot 101 automatically moves to the patient under control of the controller 110 in accordance with the work content of medical practice (for example, examination and/or test) to be performed such that a distance between the robot 101 and the patient is maintained to a predetermined first distance set in advance.

For example, in a case where the work (examination) of auscultating the front of the patient with a stethoscope is performed and then the work (examination) of auscultating the back of the patient with a stethoscope is performed, when the patient directs the back of the patient toward the robot 101, the robot 101 may automatically move backward and then automatically move to reduce a distance from the patient (maintaining the first distance).

Thus, the medical staff can operate the robot 101 by a remote operation and perform medical practice on the patient.

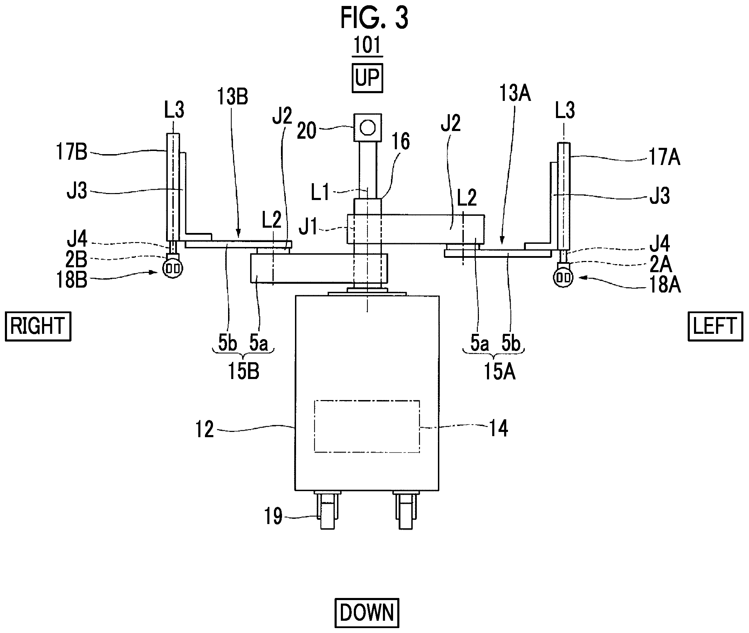

Here, the configuration of the robot 101 will be described in detail with reference to FIG. 3. A horizontal articulated dual-arm robot will be described below as the robot 101. Another robot such as a horizontal articulated robot or a vertical articulated robot may be adopted as the robot 101.

FIG. 3 is a schematic diagram illustrating a schematic configuration of the robot in the robot system illustrated in FIGS. 1 and 2. In FIG. 3, an up-down direction in the robot is represented as an up-down direction in FIG. 3.

As illustrated in FIG. 3, the robot 101 includes a carriage 12, a first arm 13A, a second arm 13B, a first hand 18A, a second hand 18B, and a controller 14 disposed in the carriage 12.

When the first arm 13A and the second arm 13B are not distinguished from each other, the first arm 13A and the second arm 13B are simply referred to as the arm 13. Similarly, when the first hand 18A and the second hand 18B are not distinguished from each other, the first hand 18A and the second hand 18B are simply referred to as the hand 18.

Further, in Embodiment 1, a form in which the controller 14 is disposed in the carriage 12 has been adopted. The present invention is not limited to this, and the controller 14 may be disposed outside the carriage 12. The controller 14 will be described later.

Wheels 19 are disposed on the lower surface of the carriage 12. An appropriate gear and a drive motor are connected to the wheels 19. Thus, the robot 101 can be self-propelled.

Further, a base shaft 16 and a first imager 20 are fixed to the upper surface of the carriage 12. The first imager 20 is configured to pick up an image and/or a video, and output the image information and/or video information obtained by pickup to the controller 110. As first imager 20, for example, a video camera or an X-ray image pickup device may be provided.

The first imager 20 may be configured to output the image information and/or video information obtained by pickup to the first display 103 without passing through the controller 110. Further, the first imager 20 may be gripped by an arm other than the first arm 13A and the second arm 13B.

The base shaft 16 is provided with the first arm 13A and the second arm 13B to be rotatable around a rotation axis L1 passing through the axis of the base shaft 16. Specifically, the first arm 13A and the second arm 13B are provided to have a vertical height difference. The first arm 13A and the second arm 13B are configured to be able to operate independently or in relation to each other.

The first arm 13A has a first arm portion 15A, a first wrist portion 17A, a first hand 18A, and a first mounting portion 2A. Similarly, the second arm 13B has a second arm portion 15B, a second wrist portion 17B, a second hand 18B, and a second mounting portion 2B. Since the second arm 13B is configured in the similar manner to the first arm 13A, detailed description thereof will be omitted.

In Embodiment 1, the first arm portion 15A is configured by a first link 5a and a second link 5b having a substantially rectangular parallelepiped shape. The first link 5a is provided with a rotating joint J1 at the proximal end portion and a rotating joint J2 at the distal end portion. Further, the second link 5b is provided with a linear motion joint J3 at the distal end portion.

The proximal end portion of the first link 5a is joined to the base shaft 16 via the rotating joint J1, and thus the first link 5a can rotate around the rotation axis L1 by the rotating joint J1. Further, the proximal end portion of the second link 5b is joined to the distal end portion of the first link 5a via the rotating joint J2, and the second link 5b can rotate around a rotation axis L2 by the rotating joint J2.

The first wrist portion 17A is joined to the distal end portion of the second link 5b via the linear motion joint J3 so as to be movable up and down with respect to the second link 5b. A rotating joint J4 is provided at the lower end portion of the first wrist portion 17A, and the first mounting portion 2A is provided at the lower end portion of the rotating joint J4.

The first mounting portion 2A is configured to be able to detachably attach the first hand 18A. Specifically, for example, the first mounting portion 2A includes a pair of rod members between which a distance is adjustable. The first hand 18A is sandwiched between the pair of rod members, and thus the first hand 18A can be attached to the first wrist portion 17A. As a result, the first hand 18A can rotate around a rotation axis L3 by the rotating joint J4. The distal end portion of the rod member may be bent.

The first hand 18A may be in any form as long as the first hand 18A is configured to hold a medical test instrument or a medical examination instrument. For example, as illustrated in FIGS. 1 and 3, the first hand 18A may be configured to hold a medical test instrument or a medical examination instrument by two claws. As the medical test instrument, for example, a sterilized cotton swab, various tubes such as a tube with a screw cap, a syringe, a catheter, or an endoscopy test instrument may be provided. Further, as the medical examination instrument, for example, a stethoscope or a tongue depressor may be provided.

In addition, the first hand 18A is configured to be able to hold various workpieces such as drugs, meals, and test reagents, and release the workpieces.

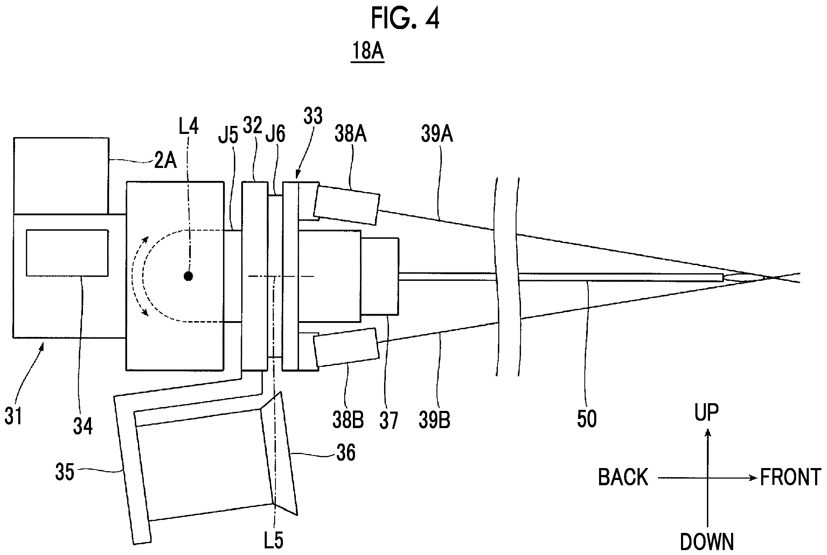

Here, another example of the first hand 18A (hand 18) will be described with reference to FIG. 4.

FIG. 4 is a schematic diagram illustrating a schematic configuration of the hand of the robot in the robot system according to Embodiment 1. In FIG. 4, an up-down direction and a front-back direction in the robot are represented as an up-down direction and a front-back direction in FIG. 4.

As illustrated in FIG. 4, the first hand 18A includes a main body 31, an intermediate member 32, and a holding member 33. The main body 31 and the intermediate member 32 are joined to each other via a rotating joint J5. Further, the intermediate member 32 and the holding member 33 are joined to each other via a rotating joint J6. As a result, the holding member 33 can rotate around a rotation axis L4 and/or a rotation axis L5 with respect to the main body 31.

The main body 31 is provided with an actuator 34 for rotating the holding member 33. The actuator 34 may be, for example, a servomotor servo-controlled by the controller 14. Further, the main body 31 is provided with a rotation sensor (not illustrated) that detects the rotation position of the servomotor and a current sensor (not illustrated) that detects a current for controlling the rotation of the servomotor. The rotation sensor may be, for example, an encoder. Position information detected by the rotation sensor and current information detected by the current sensor may be output to the controller 110 via the controller 14.

A support member 35 is provided at the lower end portion of the intermediate member 32. A camera (first imager) 36 is attached to the support member 35. The camera 36 is configured to pick up an image and/or a video and output image information and/or video information obtained by pickup to the controller 110. As the camera 36, for example, a video camera or an X-ray image pickup device may be provided.

Further, a target picked up by the camera 36 may be the nostril of the patient, for example, when the nasopharyngeal swab is collected with a sterilized cotton swab. Further, for example, when saliva or a specimen derived from the lower respiratory tract (sputum or the like) is collected by a suction catheter or the like, the oral cavity of the patient may be set as the target picked up by the camera 36.

In Embodiment 1, a form in which the support member 35 and the camera 36 are disposed at the lower end portion of the intermediate member 32 has been adopted, but the present invention is not limited to this. The support member 35 and the camera 36 may be disposed at the upper end portion of the intermediate member 32 or the like. Further, the support member 35 and the camera 36 may be disposed at the holding member 33.

A chuck mechanism 37 for holding/releasing a medical test instrument or a medical examination instrument is attached to the holding member 33. The chuck mechanism 37 may be configured by, for example, an air chuck. Here, the chuck mechanism 37 holds a sterilized cotton swab 50 for collecting a specimen for a PCR test.

Further, a pair of laser pointers (laser light indicators) 38A and 38B are arranged at the holding member 33. The laser pointers 38A and 38B are disposed so that rays of laser light 39A and 39B checked from the respective laser pointers 38A and 38B intersect with each other in front of the first hand 18A. Three or more laser light indicators may be disposed at the first hand 18A.

Thus, when the first hand 18A approaches the patient, a distance between the laser light 39A and the laser light 39B that hit the patient becomes smaller. Further, when the first hand 18A approaches the patient, the laser light that hits the patient becomes one point. Further, when the first hand 18A approaches the patient, the distance between the laser light 39A and the laser light 39B that hit the patient increases.

Therefore, the operator (medical staff) can easily understand a distance between the patient, and the distal end portion of the medical test instrument and/or the medical examination instrument (sterilized cotton swab 50) by the rays of laser light 39A and 39B with which irradiation is performed from the pair of laser pointers 38A and 38B.

Further, each of the joints J1 to J4 of the first arm 13A and the second arm 13B is provided with a drive motor as an example of an actuator that relatively rotates, or raises and lowers two members joined to each other by each joint (not illustrated). The drive motor may be, for example, a servomotor servo-controlled by the controller 14. Further, each of the joints J1 to J4 is provided with a rotation sensor (not illustrated) that detects the rotation position of the drive motor, and a current sensor (not illustrated) that detects the current for controlling the rotation of the drive motor. The rotation sensor may be, for example, an encoder. Position information detected by the rotation sensor and current information detected by the current sensor may be output to the controller 110 via the controller 14.

The controller 14 includes an arithmetic processor and a storage (not illustrated). The arithmetic processor is configured by a microprocessor, a CPU, and the like. The arithmetic processor controls various operations of the robot 101 by reading and executing software such as a basic program stored in the storage.

The storage stores types of information such as basic programs and various types of fixed data. For example, map information in the hospital may be stored in the storage in advance.

The storage does not have to be single and may be configured as a plurality of storages (for example, random access memory and hard disk drive). When the arithmetic processor is configured by a microcomputer, at least a portion of the storage may be configured as an internal memory of the microcomputer or may be configured as an independent memory.

Further, the controller 14 may control various operations of the robot 101 based on various types of command information input from the controller 110.

As illustrated in FIGS. 1 and 2, the controller 110 includes an arithmetic processor 110a, a storage 110b, and an input machine (operator) 110c. The arithmetic processor 110a is configured by a microprocessor, a CPU, and the like. The arithmetic processor 110a controls various operations of the robot system 100 by reading and executing software such as a basic program stored in the storage 110b.

The storage 110b stores types of information such as basic programs and various types of fixed data. The storage 110b does not have to be single and may be configured as a plurality of storages (for example, random access memory and hard disk drive). When the arithmetic processor 110a is configured by a microcomputer, at least a portion of the storage 110b may be configured as an internal memory of the microcomputer or may be configured as an independent memory.

The input machine 110c is capable of inputting various parameters related to the control of the robot system 100, other types of data, or the like to the arithmetic processor 110a. The input machine 110c is configured by a known input device such as a keyboard, a touch panel, and a button switch group. In Embodiment 1, for example, the position information of the patient may be set to be inputtable by the input machine 110c. Further, the position information of the patient may be inputtable by the operator 102.

The controller 110 may be configured by a single controller 110 for centralized control, or may be configured by a plurality of controllers 110 that cooperate with each other to perform distributed control. Further, the controller 110 may be configured by a microcomputer, or may be configured by an MPU, a programmable logic controller (PLC), a logic circuit, or the like.

### [Operations, and Actions and Effects of Robot System]

Next, the operation and the action and effect of the robot system 100 according to Embodiment 1 will be described in detail with reference to FIGS. 1 to 10. The following operations are performed by the arithmetic processor 110a of the controller 110 reading the program stored in the storage 110b.

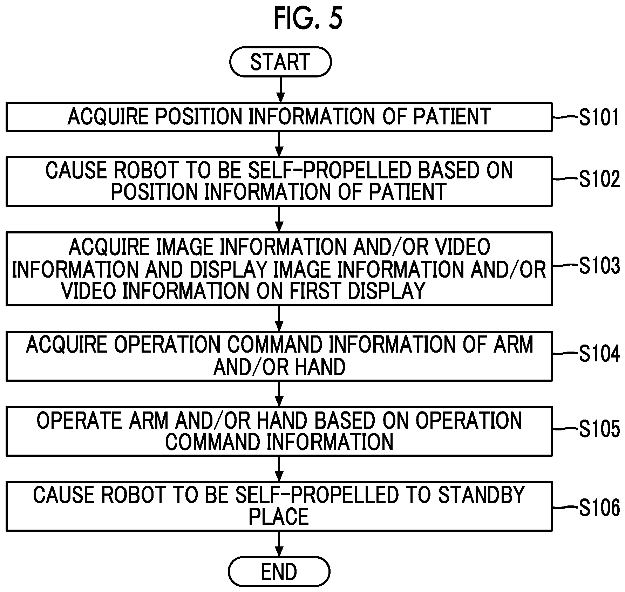

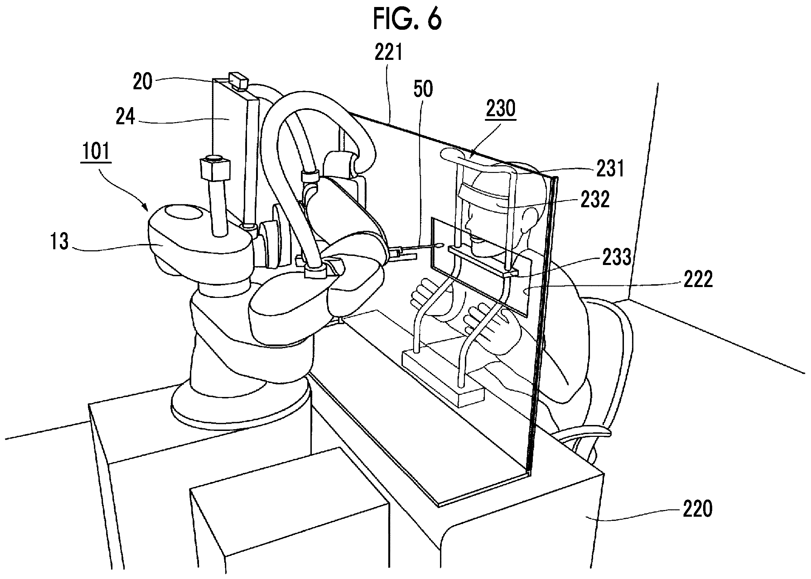

FIG. 5 is a flowchart illustrating an example of the operation of the robot system according to Embodiment 1. FIG. 6 is a schematic diagram illustrating an example of the operation of the robot in the robot system according to Embodiment 1.

As illustrated in FIG. 5, when the operator operates the input machine 110c (and/or the operator 102), the controller 110 acquires the position information of a patient from the input machine 110c (and/or the operator 102) (Step S101).

Then, the controller 110 causes the robot 101 to be self-propelled (automatically moved) from a standby place set in advance to the vicinity of the patient based on the position information of the patient acquired in Step S101 (Step S102).

Specifically, the controller 110 outputs the position information of the patient acquired in Step S101 to the controller 14. The controller 14 drives the drive motor based on the input position information of the patient and the map information in the hospital stored in the storage, and causes the robot 101 to be self-propelled to the vicinity of the patient.

As the standby place, a place (space) isolated from the first space 201 and the second space 202 may be provided.

Then, the controller 110 acquires image information and/or video information obtained by picking-up of the first imager 20 and displays the acquired image information and/or video information on the first display 103 (Step S103). The controller 110 may execute the process of Step S103 before the process of Step S101 or Step S102.

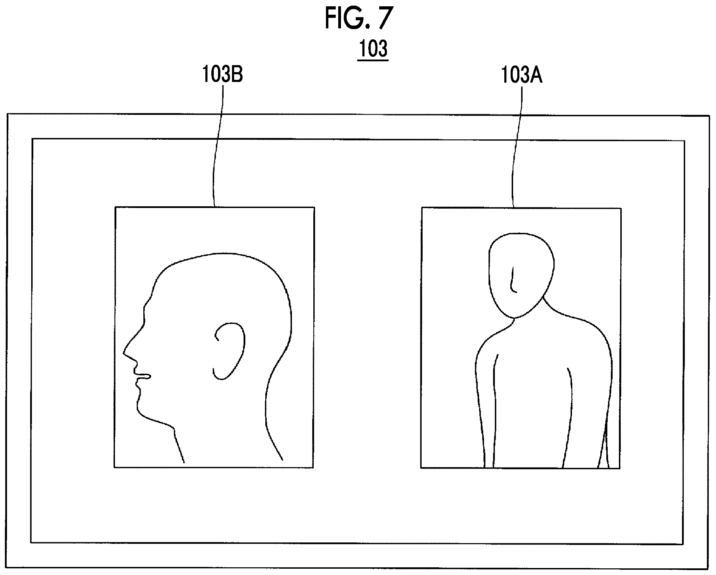

Here, the image information and/or the video information displayed on the first display 103 will be described with reference to FIGS. 7 to 10.

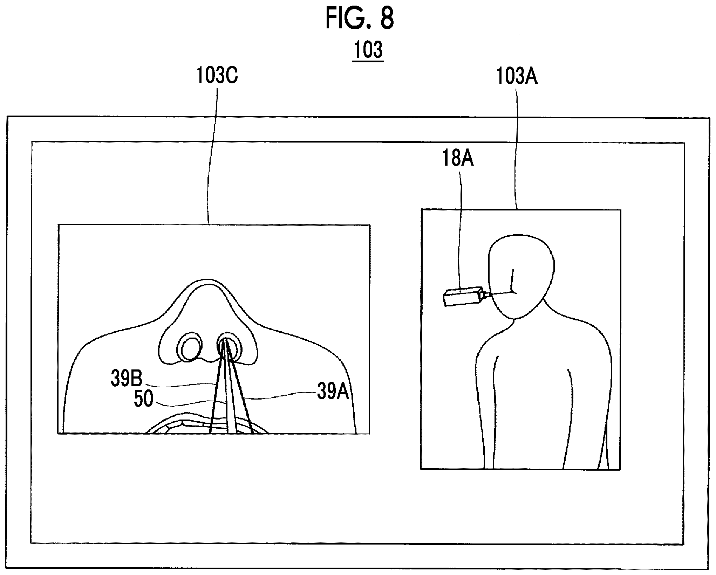

FIG. 7 is a schematic diagram illustrating an example of image information and/or video information displayed on the first display illustrated in FIG. 1. FIGS. 8 to 10 are schematic diagrams illustrating examples of the image information and/or the video information displayed on the first display illustrated in FIG. 1. In FIGS. 8 to 10, illustrations of some portions of the robot 101 and the first hand 18A are omitted.

As illustrated in FIG. 7, video information obtained by picking-up of the first imager 20 (video information obtained by picking up an image of the front of the patient) may be displayed on the first display 103 as first video information 103A. Further, video information obtained by picking-up of an image pickup device (not illustrated) that picks up the profile of the patient may be displayed on the first display 103 as second video information 103B.

Further, as illustrated in FIG. 8, video information obtained by picking-up of the first imager 20 may be displayed on the first display 103 as the first video information 103A. Further, video information obtained by picking-up of the camera 36 provided at the first hand 18A may be displayed on the first display 103 as third video information 103C.

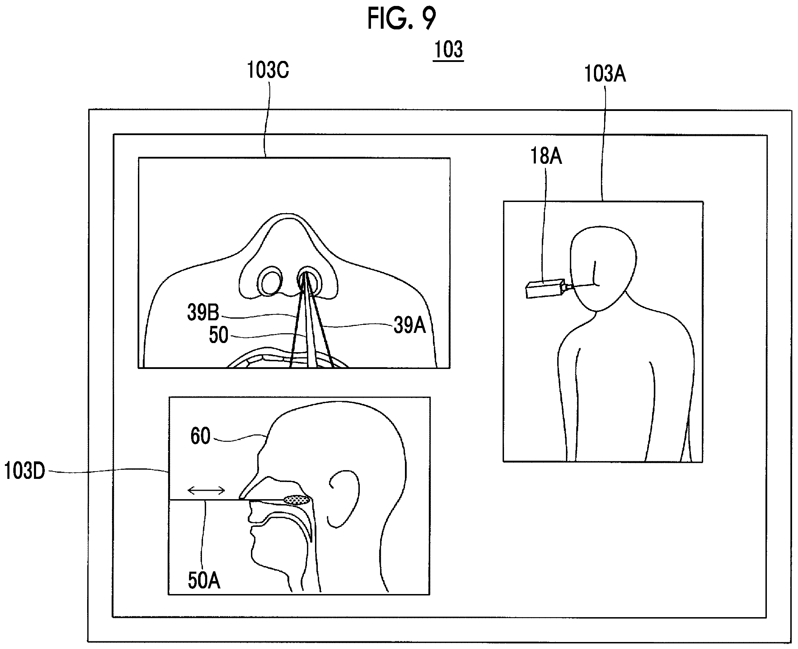

Further, as illustrated in FIG. 9, video information obtained by picking-up of the first imager 20 may be displayed on the first display 103 as the first video information 103A. Further, video information obtained by picking-up of the camera 36 provided at the first hand 18A may be displayed on the first display 103 as third video information 103C.

Further, a virtual model showing the position information of the medical test instrument and/or the medical examination instrument may be displayed on the first display 103 as the fourth video information 103D. Specifically, a virtual sterilized cotton swab 50A, which is a virtual model of the sterilized cotton swab 50, and a virtual patient 60, which is a virtual model of the medical target site of the patient, are displayed as the fourth video information 103D.

At this time, the controller 110 may move the virtual sterilized cotton swab 50A in the fourth video information 103D based on the position information of the patient, and position information detected by a rotation sensor that detects a rotation position of each drive motor and/or operation information input to the operator 102. This makes it possible for the operator to easily understand the distance between the patient, and the distal end portion of the medical test instrument and/or the medical examination instrument (sterilized cotton swab 50) .

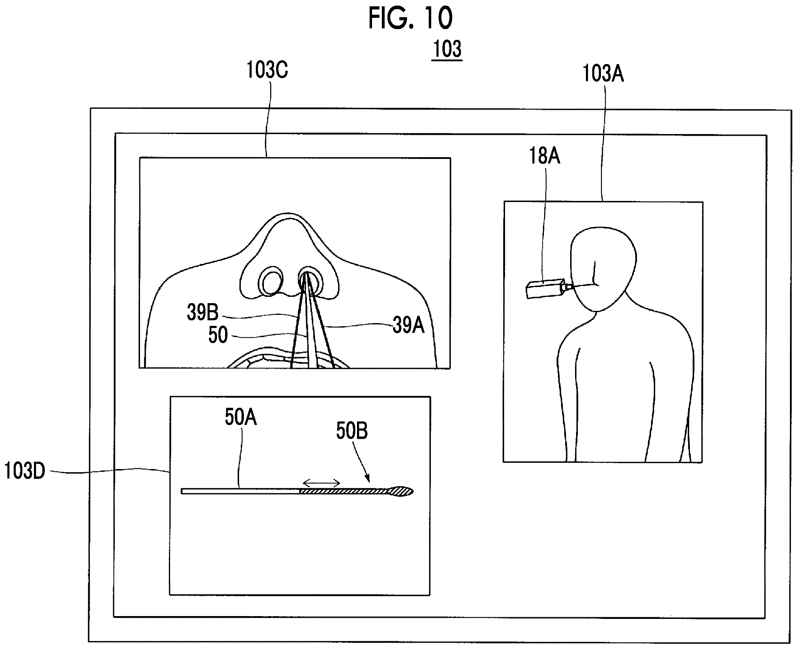

In addition, as illustrated in FIG. 10, video information obtained by picking-up of the first imager 20 may be displayed on the first display 103 as the first video information 103A. Further, video information obtained by picking-up of the camera 36 provided at the first hand 18A may be displayed on the first display 103 as third video information 103C.

Further, a virtual model showing the position information of the medical test instrument and/or the medical examination instrument may be displayed on the first display 103 as the fourth video information 103D. Specifically, as the fourth video information 103D, a virtual sterilized cotton swab 50A, which is a virtual model of the sterilized cotton swab 50, is displayed.

At this time, the controller 110 may display a region of the sterilized cotton swab 50 put into the body of the patient, as a first region 50B in the fourth video information 103D, based on the position information of the patient, and the position information detected by the rotation sensor that detects the rotation position of each drive motor and/or the operation information input to the operator 102. The first region 50B may be indicated by hatching, for example, as illustrated in FIG. 10, or may be shown in a different color than the virtual sterilized cotton swab 50A.

This makes it possible for the operator to easily understand the distance between the patient, and the distal end portion of the medical test instrument and/or the medical examination instrument (sterilized cotton swab 50) .

Then, as illustrated in FIG. 5, the controller 110 acquires the operation command information of the arm 13 and/or the hand 18 from the operator 102 (Step S104). Then, the controller 110 operates the arm 13 and/or the hand 18 based on the operation command information acquired in Step S104 (Step S105).

As a result, the operator can operate the robot 101 by a remote operation to perform medical practice (for example, examination and/or test) on the patient. For example, the operator may perform work of collecting a specimen for a PCR test from the patient.

Here, the medical practice work by the robot 101 will be described with reference to FIG. 6. In FIG. 6, a form in which the robot 101 includes a second display 24, which will be described later is adopted.

As illustrated in FIG. 6, a shielding plate 221 is disposed between the robot 101 and the patient. The shielding plate 221 may be installed on a base 220 such as a desk. Further, the shielding plate 221 is configured by a transparent member such as a glass plate, and is provided with an opening 222 in a substantially central portion.

The position and size of the opening 222 are appropriately set in accordance with the type of medical practice. For example, when medical practice for internal medicine, otolaryngology, or the like is performed, the disposition position and size of the opening 222 are appropriately set so that the mouth and the nose (medical practice target portion) of the patient are located at the opening 222. Further, when medical practice related to ophthalmology is performed, the disposition position and size of the opening 222 are appropriately set so that the eye (medical practice target portion) of the patient is located at the opening 222.

As a result, it is possible to suppress an occurrence of a situation in which droplets adhere to the robot 101 when the patient coughs or sneezes.

Further, a positioning device 230 is installed between the shielding plate 221 and the patient. The positioning device 230 includes a main body 231, an abutting target portion 232, and a chin rest 233. The main body 231 may be configured such that the patient can grip the main body 231. Further, the chin rest 233 may be configured to move up and down.

The positioning device 230 is configured such that the patient abuts the forehead on the abutting target portion 232 and puts the chin on the chin rest 233, thereby the medical practice target portion of the patient is positioned within a range (opening 222) set in advance. This facilitates the positioning of the medical practice target portion of the patient, and thus makes it possible to reduce the burden on the operation of the operator.

When executing the process of Step S105, the controller 110 may automatically operate the arm 13 and/or the hand 18 so that, for example, the distal end portion of the medical test instrument or medical examination instrument held by the hand 18 approaches the patient.

Further, the controller 110 may store the operation command information of the arm 13 and/or the hand 18 input from the operator 102, in the storage 110b. Further, the controller 110 may operate the arm 13 and/or the hand 18 based on the operation command information stored in the storage 110b, to perform medical practice (for example, examination work and/or test work) on the patient.

Further, the controller 110 may be configured to learn the examination work and the like. Specifically, for example, when the controller 110 causes the robot 101 to perform examination work or the like, in a case where the operator operates the operator 102 to correct the operation of the arm 13 and/or the hand 18, the controller 110 stores the corrected operation command information of the arm 13 and/or the hand 18 in the storage 110b.

Then, the controller 110 operates the arm 13 and/or the hand 18 based on the corrected operation command information to perform medical practice (for example, examination work and/or test work) on the patient. Then, when the operation of the arm 13 and/or the hand 18 is corrected again by the operator, the controller 110 stores the corrected operation command information of the arm 13 and/or the hand 18 in the storage 110b, and learns the examination work and the like.

Then, when the operator operates the operator 102 (and/or the input machine 110c), and thus a medical practice termination command is input from the operator 102 (and/or the input machine 110c), the controller 110 causes the robot 101 to be self-propelled to the standby place (Step S106), and then terminates this program.

The controller 110 may control the robot 101 to be in a standby state after the robot 101 is self-propelled to the standby place and then disinfected by appropriate means. Further, the robot 101 may be disinfected by a worker wearing a protective mask and protective clothing.

In the robot system 100 according to Embodiment 1, which is configured as described above, the robot 101 is configured to be self-propelled to the vicinity of the patient only by the operator (medical staff) inputting the position information of the patient. As a result, the operator can concentrate on the medical practice, and thus it is possible to reduce the burden of the operation of the operator.

The robot system 100 according to Embodiment 1 is configured so that the operator operates the robot 101 in the second space 202 isolated from the patient.

This makes it possible to suppress a contact with a patient suspected of being infected with a virus or the like. Therefore, it is possible to sufficiently suppress the infection to the operator with a virus or the like.

Further, since the robot 101 moves to the vicinity of the patient in each hospitalization room or the like, the operator does not need to move in the hospital. Therefore, it is possible to sufficiently suppress the infection to the operator.

Further, since the robot 101 moves, it is possible to reduce the number of movements and/or the movement distance of the patient infected with a virus or the like in the hospital. This makes it possible to reduce the spread of viruses and the like.

Further, in the robot system 100 according to Embodiment 1, the pair of laser pointers 38A and 38B are disposed at the first hand 18A (hand 18) so that the rays of laser light 39A and 39B with which irradiation is respectively performed by the pair of laser pointers 38A and 38B intersect with each other.

Thus, when the first hand 18A approaches the patient, a distance between the laser light 39A and the laser light 39B that hit the patient becomes smaller. Further, when the first hand 18A approaches the patient, the laser light that hits the patient becomes one point. Further, when the first hand 18A approaches the patient, the distance between the laser light 39A and the laser light 39B that hit the patient increases.

Therefore, the operator (medical staff) can easily understand a distance between the patient, and the distal end portion of the medical test instrument and/or the medical examination instrument (sterilized cotton swab 50) by the rays of laser light 39A and 39B with which irradiation is performed from the pair of laser pointers 38A and 38B.

Further, in the robot system 100 according to Embodiment 1, the virtual model showing the position information of the medical test instrument and/or the medical examination instrument is displayed on the first display 103 as the fourth video information 103D. This makes it possible for the operator to easily understand the distance between the patient, and the distal end portion of the medical test instrument and/or the medical examination instrument (sterilized cotton swab 50).

At this time, the controller 110 displays the virtual patient 60, which is a virtual model of the medical target site of the patient, on the first display 103, thereby it is possible to more easily understand the distance between the patient, and the distal end portion of the medical test instrument and/or the medical examination instrument (sterilized cotton swab 50).

### [Modification Example 1]

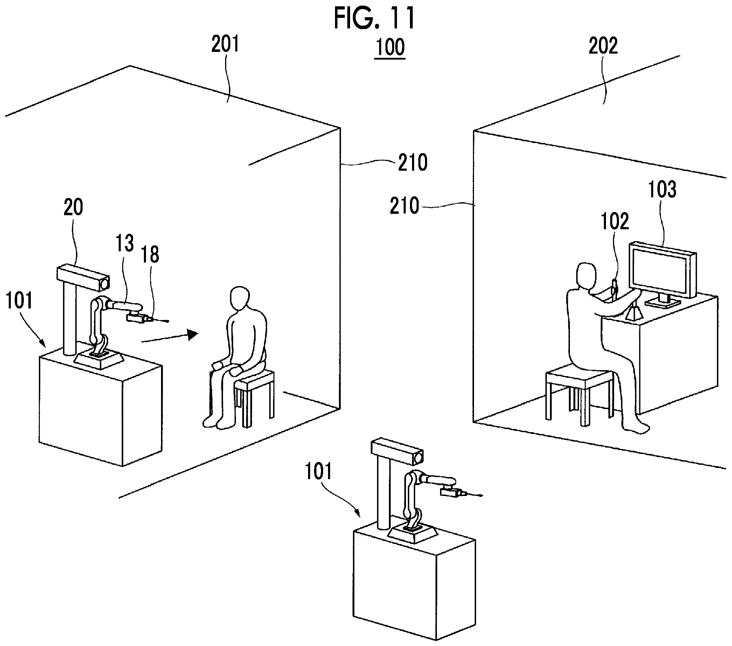

Next, a modification example of the robot system 100 according to Embodiment 1 will be described below with reference to FIG. 11.

FIG. 11 is a schematic diagram illustrating a schematic configuration of a robot system according to Modification Example 1 in Embodiment 1.

As illustrated in FIG. 11, a robot system 100 in Modification Example 1 has the same basic configuration as the robot system 100 according to Embodiment 1, but Modification Example 1 is different from Embodiment 1 in that a robot 101 is configured by a vertical articulated robot.

The robot system 100 in Modification Example 1, which is configured as described above, also has the similar action and effect to those of the robot system 100 according to Embodiment 1.

### (Embodiment 2)

In a robot system according to Embodiment 2, a robot further includes a first audio input and a first audio output in the robot system according to Embodiment 1 (including the modification example). In addition, a second audio input and a second audio output are further disposed in a second space. A controller is configured to output audio information input to the first audio input, to the second audio output, and output audio information input to the second audio input, to the first audio output.

In a method for operating a robot system according to Embodiment 2, a robot further includes a first audio input and a first audio output in the method for operating the robot system according to Embodiment 1 (including the modification example). In addition, a second audio input and a second audio output are further disposed in a second space. A controller is configured to output audio information input to the first audio input, to the second audio output, and output audio information input to the second audio input, to the first audio output.

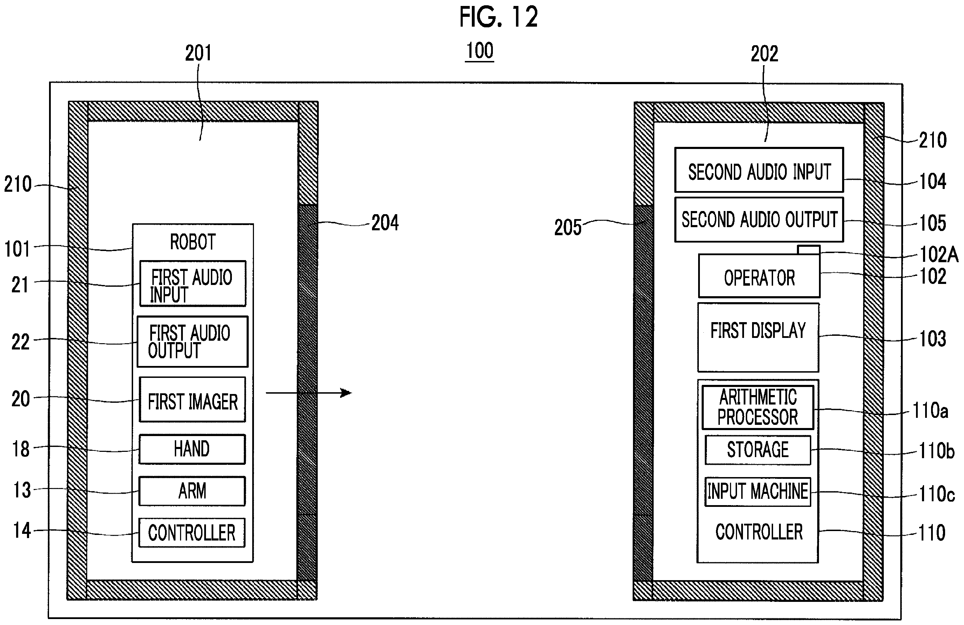

An example of the robot system according to Embodiment 2 will be described below with reference to FIG. 12.

### [Configuration of Robot System]

FIG. 12 is a schematic diagram illustrating a schematic configuration of the robot system according to Embodiment 2.

As illustrated in FIG. 12, the robot system 100 according to Embodiment 2 has the same basic configuration as the robot system 100 according to Embodiment 1. Embodiment 2 is different from Embodiment 1 in that the robot 101 includes a first audio input 21 and a first audio output 22, and that a second audio input 104 and a second audio output 105 are disposed in the second space 202.

The first audio input 21 and the second audio input 104 may be configured, for example, by a microphone. In addition, the first audio output 22 and the second audio output 105 may be configured by a speaker.

The second audio input 104 and the second audio output 105 may be configured by headphones (headsets) with a microphone. In addition, when the first display 103 is configured by a head-mounted display, the second audio input 104 and the second audio output 105 may be configured by a microphone and headphones attached to the head-mounted display.

The robot system 100 in Embodiment 2, which is configured as described above, also has the similar action and effect to those of the robot system 100 according to Embodiment 1.

Further, in the robot system 100 according to Embodiment 2, the robot 101 is provided with the first audio input 21 and the first audio output 22, and the second audio input 104 and the second audio output 105 are disposed in the second space 202. Thus, it is possible to perform communication between the patient and the operator.

As a result, for example, when the operator is a medical staff, or when there is a medical staff beside the operator, it is possible to perform, on the patient, medical practice such as inquiries, auscultation, transmission of test results, communication of treatment policies, and the like.

### (Embodiment 3)

In a robot system according to Embodiment 3, a robot further includes a container that contains at least one transfer item among a drug, a meal, a test reagent, a specimen, a medical test instrument, and a medical examination instrument in the robot system according to Embodiment 1(including the modification example) or Embodiment 2.

In a method for operating a robot system according to Embodiment 3, a robot further includes a container that contains at least one transfer item among a drug, a meal, a test reagent, a specimen, a medical test instrument, and a medical examination instrument in the method for operating the robot system according to Embodiment 1(including the modification example) or Embodiment 2.

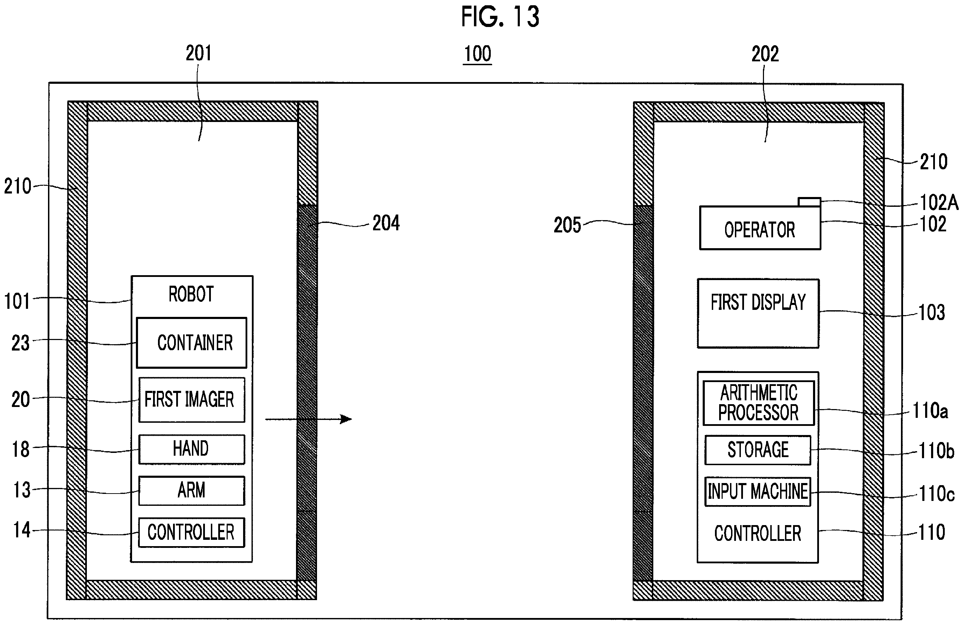

An example of the robot system according to Embodiment 3 will be described below with reference to FIG. 13.

### [Configuration of Robot System]

FIG. 13 is a schematic diagram illustrating a schematic configuration of the robot system according to Embodiment 3.

As illustrated in FIG. 13, a robot system 100 according to Embodiment 3 has the same basic configuration as the robot system 100 according to Embodiment 1. Embodiment 3 is different from Embodiment 1 in that a robot 101 further includes a container 23 that contains at least one transfer item among a drug, a meal, a test reagent, a specimen, a medical test instrument, and a medical examination instrument.

As the container 23, various containment items such as a box with a lid and a tray can be used. Further, the container 23 may be made of metal (for example, stainless steel) so as to be capable of supporting a sterilization process such as autoclave sterilization and dry heat sterilization. Further, the container 23 may be configured so that the internal space can be maintained at a predetermined temperature (for example, 0°C, -20°C, or - 80°C) in order to be able to transfer a specimen.

Further, various instruments and/or experimental devices such as an autopipette, a tip used for the autopipette, a microtube, a centrifuge settling tube, a centrifuge, and a PCR device may be contained in the container 23.

The robot system 100 in Embodiment 3, which is configured as described above, also has the similar action and effect to those of the robot system 100 according to Embodiment 1.

Further, in the robot system 100 according to Embodiment 3, the robot 101 further includes the container 23 that contains at least one transfer item among a drug, a meal, a test reagent, a specimen, a medical test instrument, and a medical examination instrument. This makes it possible to reduce the work of medical practice assistants such as nurses. In addition, it is possible to reduce the chance of the medical practice assistant coming into contact with a patient with an infectious disease such as a virus. Thus, it is possible to sufficiently suppress the infection of the medical practice assistant with a virus or the like.

### (Embodiment 4)

In a robot system according to Embodiment 4, a third space isolated from the first space and the second space is further provided in the robot system according to any of Embodiment 1 (including the modification example) to Embodiment 3. In addition, a robot is disinfected in the third space.

Further, in the robot system according to Embodiment 4, the robot may be configured to disinfect the robot itself.

Further, in the robot system according to Embodiment 4, a controller may be configured to further (C) cause the robot to be self-propelled to the third space and disinfect the robot, after (B).

In a method for operating a robot system according to Embodiment 4, a third space isolated from the first space and the second space is further provided in the method for operating the robot system according to any of Embodiment 1 (including the modification example) to Embodiment 3. In addition, a robot is disinfected in the third space.

Further, in the method for operating the robot system according to Embodiment 4, the robot may be configured to disinfect the robot itself.

Further, the method for operating the robot system according to Embodiment 4 may further include (C) in which the robot is self-propelled to the third space and disinfects the robot after (B).

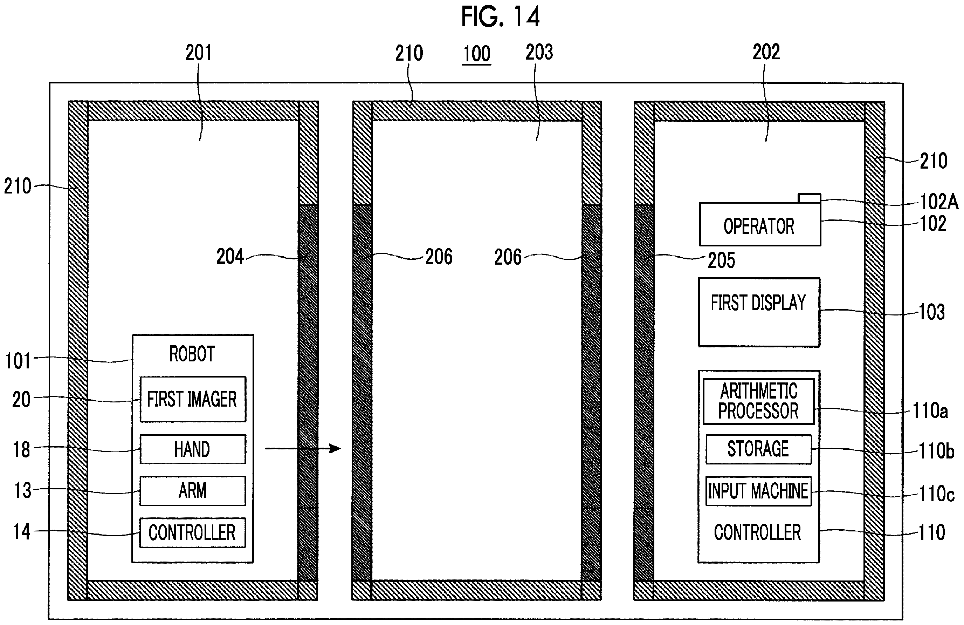

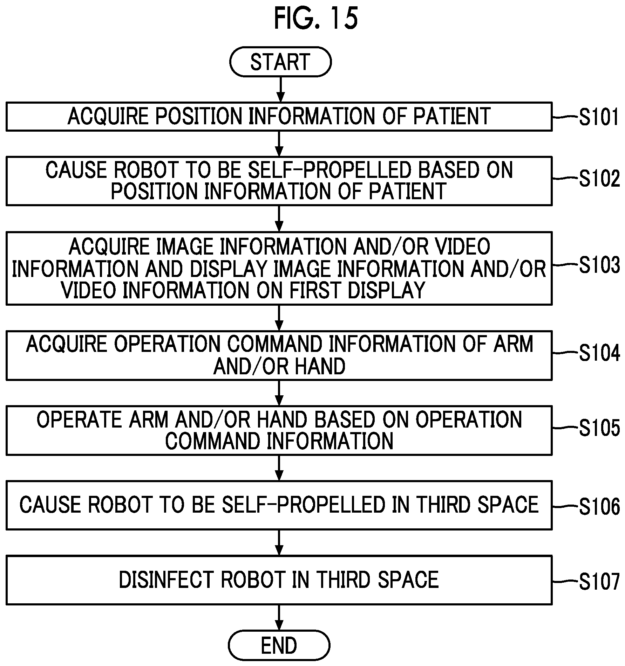

An example of the robot system according to Embodiment 4 will be described below with reference to FIGS. 14 and 15.

### [Configuration of Robot System]

FIG. 14 is a schematic diagram illustrating a schematic configuration of the robot system according to Embodiment 4.

As illustrated in FIG. 14, a robot system 100 according to Embodiment 4 has the same basic configuration as the robot system 100 according to Embodiment 1. Embodiment 4 is different from Embodiment 1 in that a third space 203 isolated from the first space 201 and the second space 202 are further provided.

The first space 201, the second space 202, and the third space 203 are spaces separated from each other. The first space 201, the second space 202, and the third space 203 are separated by a partition wall member 210, respectively.

An anterior chamber may be provided in a room (sterility chamber) constituting the third space 203. Further, a fan filter unit that makes the anterior chamber have negative pressure and makes the second space 202 (internal space of the sterility chamber) have positive pressure may be installed in the anterior chamber. A known fan filter unit can be used as the fan filter unit.

The partition wall member 210 may be provided with a shutter (door) 206 that permits/prohibits movement into the third space 203.

The robot 101 may be configured to disinfect the robot 101 itself. Specifically, the robot 101 may be disinfected by itself, for example, in a manner that a sprayer for spraying a solution such as an ethanol solution having sterilizing and antiviral effects is held with the hand 18 and the solution is sprayed toward the robot 101.

Further, the robot 101 may be disinfected by itself in a manner that an irradiator for irradiation with ultraviolet rays is held with the hand 18, and the robot 101 is irradiated with the ultraviolet rays.

Further, a protective cover (surgical drape) may be disposed in the third space 203. The robot 101 may be configured to maintain a sterilized/antiviral state by detachably attaching the protective cover.

Specifically, the robot 101 wears the protective cover in the third space 203, and then moves into the first space 201 to perform medical practice. After the medical practice is terminated, the robot 101 moves into another third space 203 in which the protective cover is not disposed, and then removes the protective cover. Then, the robot 101 moves into the third space 203 in which the protective cover is disposed, and wears the protective cover.

### [Operations, and Actions and Effects of Robot System]

Next, the operation and the action and effect of the robot system 100 according to Embodiment 4 will be described in detail with reference to FIGS. 14 to 15. The following operations are performed by the arithmetic processor 110a of the controller 110 reading the program stored in the storage 110b.

FIG. 15 is a flowchart illustrating an example of the operation of the robot system according to Embodiment 4.

As illustrated in FIG. 15, the operation of the robot system 100 according to Embodiment 4 is basically the same as that of the robot system 100 according to Embodiment 1. Embodiment 4 is different from Embodiment 1 in that the controller 110 executes a process of Step S106A instead of the process of Step S106, and executes a process of Step S107 after the process of Step S106A.

Specifically, when medical practice termination command information is input from the operator 102 (and/or the input machine 110c), the controller 110 causes the robot 101 to be self-propelled to the third space 203 (Step S106A).

Then, the controller 110 disinfects the robot 101 in the third space 203 (Step S107), and terminates this program.

The robot system 100 in Embodiment 4, which is configured as described above, also has the similar action and effect to those of the robot system 100 according to Embodiment 1.

Further, in the robot system 100 according to Embodiment 4, the robot 101 is configured to disinfect the robot 101 itself. This eliminates the need for the worker wearing the protective mask and the protective clothing to disinfect the robot 101. Therefore, it is possible to provide an easy-to-use robot system 100.

### [Modification Example 1]

Next, a modification example of the robot system 100 according to Embodiment 4 will be described.

In the robot system according to Modification Example 1 in Embodiment 4, a disinfector configured to disinfect the robot is disposed in the third space.

In a method for operating a robot system according to Modification Example 1 in Embodiment 4, a disinfector configured to disinfect the robot is disposed in the third space.

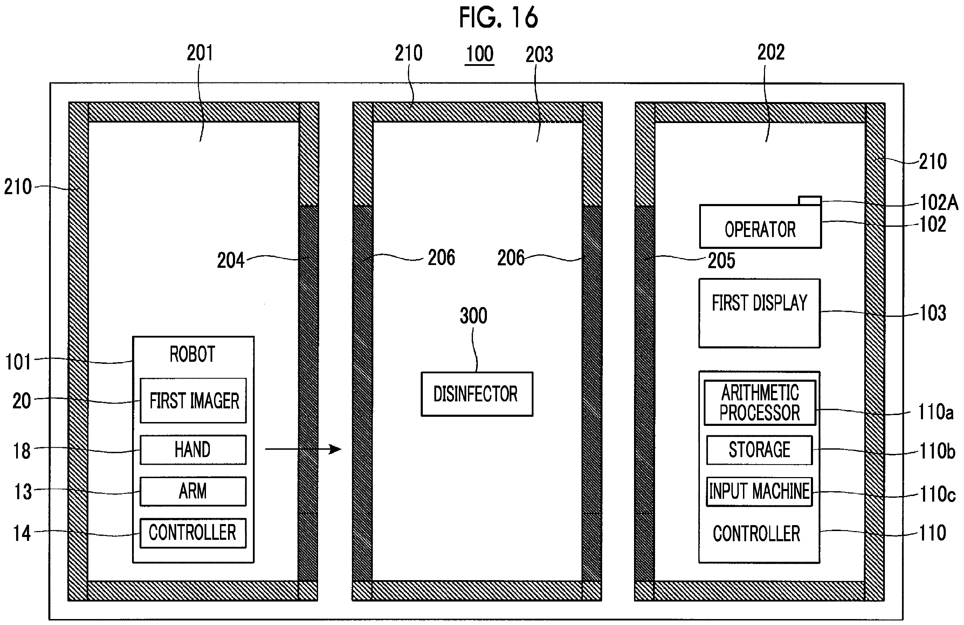

An example of the robot system according to Modification Example 1 in Embodiment 4 will be described below with reference to FIG. 16.

### [Configuration of Robot System]

FIG. 16 is a schematic diagram illustrating a schematic configuration of the robot system according to Modification Example 1 in Embodiment 4.

As illustrated in FIG. 16, the robot system 100 in Modification Example 1 has the same basic configuration as that of the robot system 100 according to Embodiment 4. Modification Example 1 is different from Embodiment 4 in that a disinfector 300 is disposed in a sterility chamber constituting the third space 203.

As the disinfector 300, a sprayer that sprays a solution such as an ethanol solution having sterilizing and antiviral effects may be provided. Further, as the disinfector 300, an irradiator that performs irradiation with ultraviolet rays may be provided. Further, a robot different from the robot 101 may be disposed in the sterility chamber, and the robot may hold the sprayer or the irradiator to perform the disinfection work on the robot 101.

The robot system 100 in Modification Example 1, which is configured as described above, also has the similar action and effect to those of the robot system 100 according to Embodiment 4.

### (Embodiment 5)

In a robot system according to Embodiment 5, a robot further includes a second display in the robot system according to any of Embodiment 1 to Embodiment 4 (including the modification examples). In addition, a second imager is further disposed in the second space, and the controller is configured to display image information and/or video information obtained by picking-up of the second imager, on the second display in (B).

In a method for operating a robot system according to Embodiment 5, a robot further includes a second display in the method for operating the robot system according to any of Embodiment 1 to Embodiment 4 (including the modification examples). In addition, a second imager is further disposed in the second space, and, in (B), the second display is configured to display image information and/or video information obtained by picking-up of the second imager.

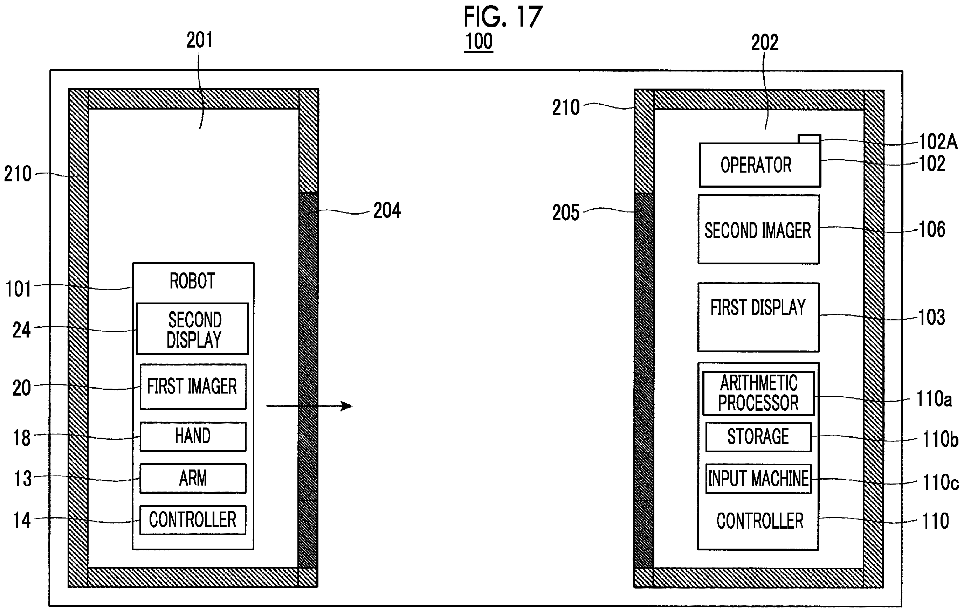

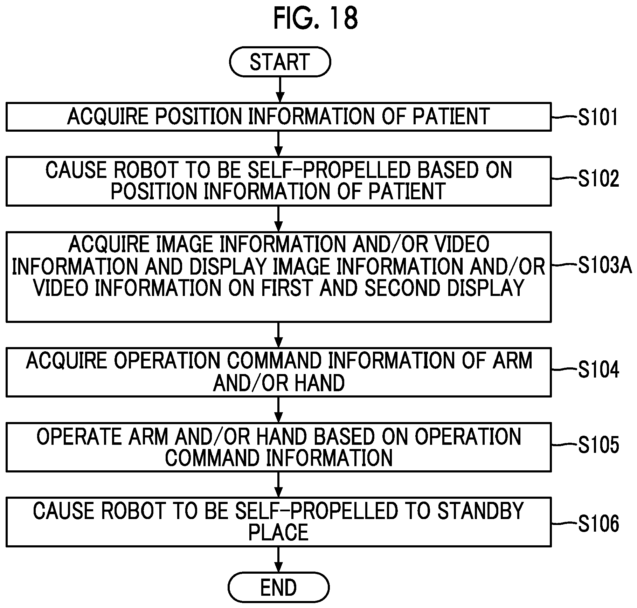

An example of the robot system according to Embodiment 5 will be described below with reference to FIGS. 17 and 18.

### [Configuration of Robot System]

FIG. 17 is a schematic diagram illustrating a schematic configuration of the robot system according to Embodiment 5.

As illustrated in FIG. 17, a robot system 100 according to Embodiment 5 has the same basic configuration as that of the robot system 100 according to Embodiment 1. Embodiment 5 is different from Embodiment 1 in that a robot 101 further includes a second display 24, and that a second imager 106 is further disposed in the second space 202.

The second display 24 is configured to display image information and/or video information obtained by picking-up of the second imager 106. For example, a stationary type display may be configured as the second display 24.

The second imager 106 is configured to pick up an image and/or a video, and output image information and/or video information obtained by pickup to the second display 24 via the controller 110 and the controller 14. For example, a video camera may be used as the second imager 106.

### [Operations, and Actions and Effects of Robot System]

Next, the operation and the action and effect of the robot system 100 according to Embodiment 5 will be described in detail with reference to FIGS. 17 to 18. The following operations are performed by the arithmetic processor 110a of the controller 110 reading the program stored in the storage 110b.

FIG. 18 is a flowchart illustrating an example of an operation of the robot system according to Embodiment 5.

As illustrated in FIG. 18, the operation of the robot system 100 according to Embodiment 5 is basically the same as that of the robot system 100 according to Embodiment 1. Embodiment 5 is different from Embodiment 1 in that the controller 110 executes a process of Step S103A instead of the process of Step S103.

Specifically, the controller 110 is self-propelled from the standby place to the vicinity of the patient (Step S102), and then executes processes as follows.

The controller 110 acquires the image information and/or the video information obtained by picking-up of the first imager 20, and displays the image information and/or the video information on the first display 103. In addition, the controller 110 acquires the image information and/or the video information obtained by picking-up of the second imager 106, and displays the image information and/or the video information on the second display 24 (Step S103A). The controller 110 may execute the process of Step S103A before the process of Step S101 or Step S102.

The robot system 100 in Embodiment 5, which is configured as described above, also has the similar action and effect to those of the robot system 100 according to Embodiment 1.

Further, in the robot system 100 according to Embodiment 5, the robot 101 further includes the second display 24, and the second imager 106 is further disposed in the second space 202.

This makes it possible to communicate between the patient and the operator (medical staff).

### (Embodiment 6)

According to Embodiment 6, a robot system includes a robot including an arm with a hand that holds a medical test instrument and/or a medical examination instrument, an operator configured to operate the robot, and a controller. A first space in which the robot is disposed is isolated from a second space in which the operator is disposed, and a first imager is disposed at the hand. The controller is configured to (α) operate the arm and/or the hand based on operation command information of the arm and/or the hand, which has been input from the operator.

Further, the robot system according to Embodiment 6 may further include a first imager and a first display configured to display image information and/or video information obtained by picking-up of the first imager. The controller may be configured to perform (α) in a state where the first display displays the image information and/or the video information obtained by picking-up of the first imager.

Further, in the robot system according to Embodiment 6, the robot and the operator may be configured by a master-slave method.

Further, in the robot system according to Embodiment 6, the first imager may be disposed at the robot, or may be disposed at the hand of the robot.

Further, in the robot system according to Embodiment 6, a pair of laser light indicators may be disposed at the hand so that rays of light with which irradiation is performed from the laser light indicators intersect with each other.

Further, in the robot system according to Embodiment 6, the first display may be configured to display a virtual model showing position information of the medical test instrument and/or the medical examination instrument.

Further, in the robot system according to Embodiment 6, the first display may be configured to display a virtual model of a medical practice target site of a patient.

Furthermore, in the robot system according to Embodiment 6, an operation switch configured to perform an instruction to release holding of the medical test instrument and/or the medical examination instrument may be disposed at the operator.

Further, in a method for operating a robot system according to Embodiment 6, a robot system includes a robot including an arm with a hand that holds a medical test instrument and/or a medical examination instrument, and an operator configured to operate the robot. A first space in which the robot is disposed is isolated from a second space in which the operator is disposed. A first imager is disposed at the hand. The method includes (α) in which the arm and/or the hand operates based on operation command information of the arm and/or the hand, which is input from the operator.

Further, in the method for operating a robot system according to Embodiment 6, the robot system may further include a first imager and a first display configured to display image information and/or video information obtained by picking-up of the first imager. (α) may be performed in a state where the image information and/or the video information obtained by picking-up of the first imager is displayed on the first display.

Further, in the method for operating a robot system according to Embodiment 6, the robot and the operator may be configured by a master-slave method.

Further, in the method for operating a robot system according to Embodiment 6, the first imager may be disposed at the robot or at the hand.

Further, in the method for operating a robot system according to Embodiment 6, a pair of laser light indicators may be disposed at the hand so that the rays of light with which irradiation is performed from the laser light indicators intersect with each other.

Further, in the method for operating a robot system according to Embodiment 6, the first display may be configured to display a virtual model showing position information of the medical test instrument and/or the medical examination instrument.

Further, in the method for operating a robot system according to Embodiment 6, the first display may be configured to display a virtual model of a medical practice target site of a patient.

Furthermore, in the method for operating a robot system according to Embodiment 6, an operation switch configured to perform an instruction to release holding of the medical test instrument and/or the medical examination instrument may be disposed at the operator.

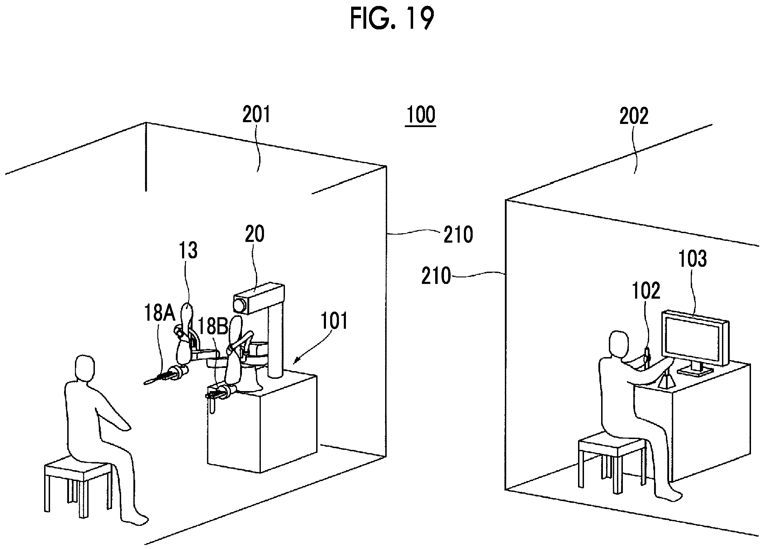

An example of the robot system according to Embodiment 6 will be described below with reference to FIGS. 19 to 22.

### [Configuration of Robot System]

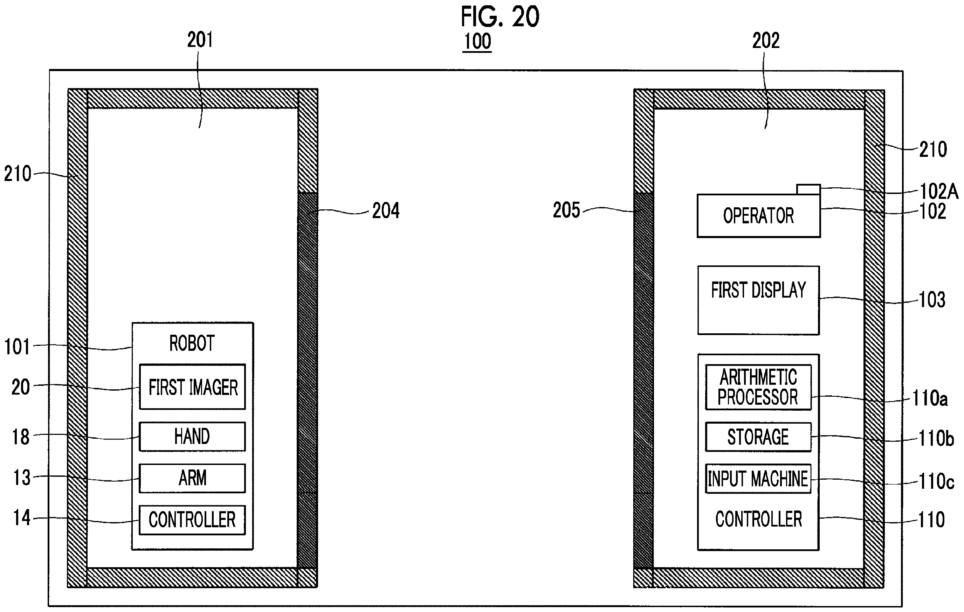

FIGS. 19 and 20 are schematic diagrams illustrating a schematic configuration of a robot system according to Embodiment 6.

As illustrated in FIGS. 19 and 20, a robot system 100 according to Embodiment 6 has the same basic configuration as that of the robot system 100 according to Embodiment 1. Embodiment 6 is different from Embodiment 1 in that a robot 101 is installed in a first space 201 (the robot 101 is a stationary type). Further, the configuration of a first hand 18A in the robot 101 is different.

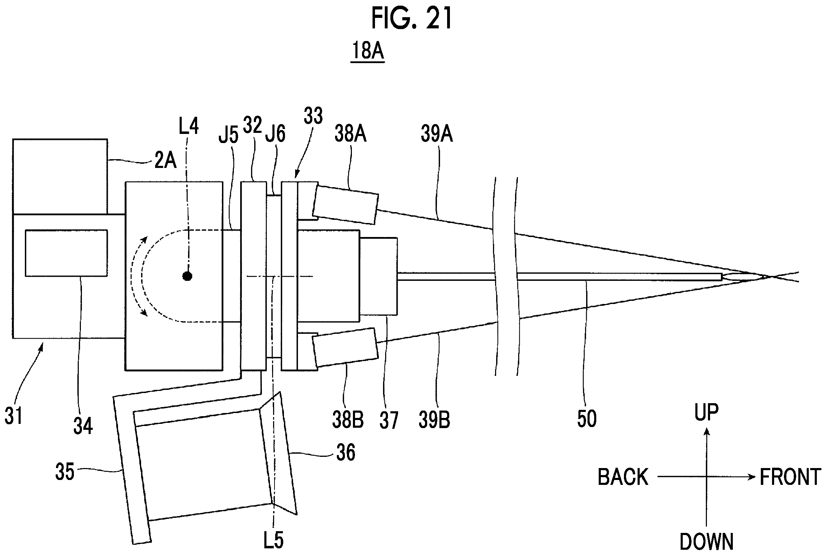

Here, the configuration of the first hand 18A of the robot 101 will be described with reference to FIG. 21.

FIG. 21 is a schematic diagram illustrating a schematic configuration of the hand of the robot illustrated in FIG. 19. In FIG. 21, an up-down direction and a front-back direction in the robot are represented as an up-down direction and a front-back direction in FIG. 21.

As illustrated in FIG. 21, the first hand 18A includes a main body 31, an intermediate member 32, and a holding member 33. The main body 31 and the intermediate member 32 are joined to each other via a rotating joint J5. Further, the intermediate member 32 and the holding member 33 are joined to each other via a rotating joint J6. As a result, the holding member 33 can rotate around a rotation axis L4 and/or a rotation axis L5 with respect to the main body 31.

The main body 31 is provided with an actuator 34 for rotating the holding member 33. The actuator 34 may be, for example, a servomotor servo-controlled by the controller 14. Further, the main body 31 is provided with a rotation sensor (not illustrated) that detects the rotation position of the servomotor and a current sensor (not illustrated) that detects a current for controlling the rotation of the servomotor. The rotation sensor may be, for example, an encoder. Position information detected by the rotation sensor and current information detected by the current sensor may be output to the controller 110 via the controller 14.

A support member 35 is provided at the lower end portion of the intermediate member 32. A camera (first imager) 36 is attached to the support member 35. The camera 36 is configured to pick up an image and/or a video and output image information and/or video information obtained by pickup to the controller 110. As the camera 36, for example, a video camera or an X-ray image pickup device may be provided.

Further, a target picked up by the camera 36 may be the nostril of the patient, for example, when the nasopharyngeal swab is collected with a sterilized cotton swab. Further, for example, when saliva or a specimen derived from the lower respiratory tract (sputum or the like) is collected by a suction catheter or the like, the oral cavity of the patient may be set as the target picked up by the camera 36.

In Embodiment 1, a form in which the support member 35 and the camera 36 are disposed at the lower end portion of the intermediate member 32 has been adopted, but the present invention is not limited to this. The support member 35 and the camera 36 may be disposed at the upper end portion of the intermediate member 32 or the like. Further, the support member 35 and the camera 36 may be disposed at the holding member 33.

A chuck mechanism 37 for holding/releasing a medical test instrument or a medical examination instrument is attached to the holding member 33. The chuck mechanism 37 may be configured by, for example, an air chuck. Here, the chuck mechanism 37 holds a sterilized cotton swab 50 for collecting a specimen for a PCR test.

Further, a pair of laser pointers (laser light indicators) 38A and 38B are arranged at the holding member 33. The laser pointers 38A and 38B are disposed so that rays of laser light 39A and 39B checked from the respective laser pointers 38A and 38B intersect with each other in front of the first hand 18A. Three or more laser light indicators may be disposed at the first hand 18A.

Thus, when the first hand 18A approaches the patient, a distance between the laser light 39A and the laser light 39B that hit the patient becomes smaller. Further, when the first hand 18A approaches the patient, the laser light that hits the patient becomes one point. Further, when the first hand 18A approaches the patient, the distance between the laser light 39A and the laser light 39B that hit the patient increases.

Therefore, the operator (medical staff) can easily understand a distance between the patient, and the distal end portion of the medical test instrument and/or the medical examination instrument (sterilized cotton swab 50) by the rays of laser light 39A and 39B with which irradiation is performed from the pair of laser pointers 38A and 38B.

### [Operations, and Actions and Effects of Robot System]

Next, the operation and the action and effect of the robot system 100 according to Embodiment 6 will be described in detail with reference to FIGS. 19 to 22. The following operations are performed by the arithmetic processor 110a of the controller 110 reading the program stored in the storage 110b.

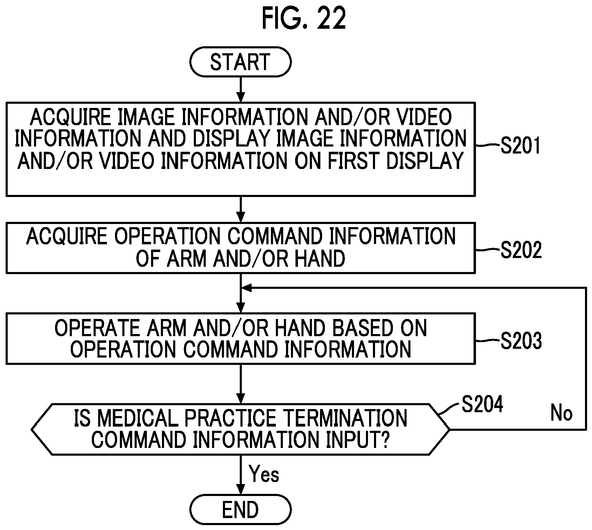

FIG. 22 is a flowchart illustrating an example of an operation of the robot system according to Embodiment 6.

As illustrated in FIG. 22, the controller 110 acquires image information and/or video information obtained by picking-up of the first imager 20 and displays the acquired image information and/or video information on the first display 103 (Step S201). The image information and/or video information displayed on the first display 103 may be the same as that in the examples illustrated in FIGS. 6 to 10.

Then, the controller 110 acquires operation command information of the arm 13 and/or the hand 18 from the operator 102 (Step S202). Then, the controller 110 operates the arm 13 and/or the hand 18 based on the operation command information acquired in Step S104 (Step S203).

As a result, the operator can operate the robot 101 by a remote operation to perform medical practice (for example, examination and/or test) on the patient (see FIG. 6). For example, the operator may perform work of collecting a specimen for a PCR test.

The controller 110 may store the operation command information of the arm 13 and/or the hand 18 input from the operator 102, in the storage 110b. Further, the controller 110 may operate the arm 13 and/or the hand 18 based on the operation command information stored in the storage 110b, to perform medical practice (for example, examination work and/or test work) on the patient.

Further, the controller 110 may be configured to learn the examination work and the like. Specifically, for example, when the controller 110 causes the robot 101 to perform examination work or the like, in a case where the operator operates the operator 102 to correct the operation of the arm 13 and/or the hand 18, the controller 110 stores the corrected operation command information of the arm 13 and/or the hand 18 in the storage 110b.

Then, the controller 110 operates the arm 13 and/or the hand 18 based on the corrected operation command information to perform medical practice (for example, examination work and/or test work) on the patient. Then, when the operation of the arm 13 and/or the hand 18 is corrected again by the operator, the controller 110 stores the corrected operation command information of the arm 13 and/or the hand 18 in the storage 110b, and learns the examination work and the like.

Then, when the operator operates the operator 102 (and/or the input machine 110c), and thus medical practice termination command information is input from the operator 102 (and/or the input machine 110c) (Yes in Step S204), the controller 110 terminates this program.

After terminating this program, the controller 110 may control the robot 101 to be in a standby state after disinfecting the robot 101 by appropriate means. Further, the robot 101 may be disinfected by a worker wearing a protective mask and protective clothing.

In the robot system 100 according to Embodiment 6, which is configured as described above, the operator operates the robot 101 in the second space 202 isolated from the patient.

This makes it possible to suppress a contact with a patient suspected of being infected with a virus or the like. Therefore, it is possible to sufficiently suppress the infection to the operator with a virus or the like.

Further, in the robot system 100 according to Embodiment 6, the pair of laser pointers 38A and 38B are disposed at the first hand 18A (hand 18) so that the rays of laser light 39A and 39B with which irradiation is respectively performed by the pair of laser pointers 38A and 38B intersect with each other.

Thus, when the first hand 18A approaches the patient, a distance between the laser light 39A and the laser light 39B that hit the patient becomes smaller. Further, when the first hand 18A approaches the patient, the laser light that hits the patient becomes one point. Further, when the first hand 18A approaches the patient, the distance between the laser light 39A and the laser light 39B that hit the patient increases.

Therefore, the operator (medical staff) can easily understand a distance between the patient, and the distal end portion of the medical test instrument and/or the medical examination instrument (sterilized cotton swab 50) by the rays of laser light 39A and 39B with which irradiation is performed from the pair of laser pointers 38A and 38B.

Further, in the robot system 100 according to Embodiment 6, the virtual model showing the position information of the medical test instrument and/or the medical examination instrument is displayed on the first display 103 as the fourth video information 103D. This makes it possible for the operator to easily understand the distance between the patient, and the distal end portion of the medical test instrument and/or the medical examination instrument (sterilized cotton swab 50).

At this time, the controller 110 displays the virtual patient 60, which is a virtual model of the medical target site of the patient, on the first display 103, thereby it is possible to more easily understand the distance between the patient, and the distal end portion of the medical test instrument and/or the medical examination instrument (sterilized cotton swab 50).

### [Modification Example 1]

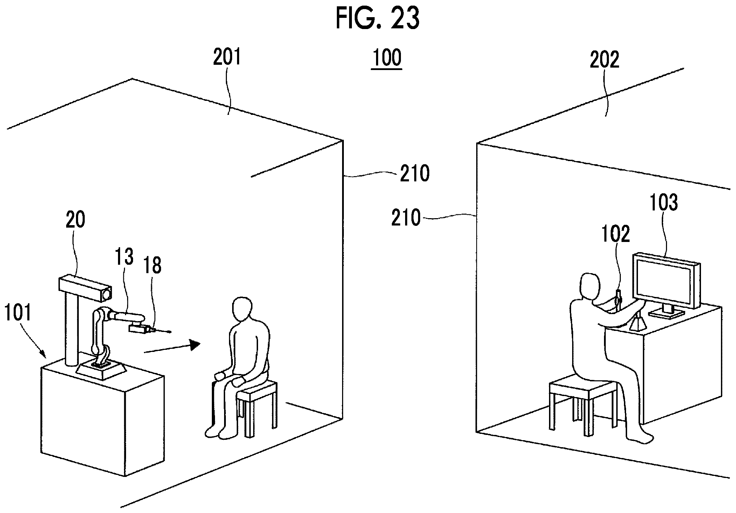

Next, a modification example of the robot system according to Embodiment 6 will be described below with reference to FIG. 23.

FIG. 23 is a schematic diagram illustrating a schematic configuration of the robot system according to Modification Example 1 in Embodiment 6.

As illustrated in FIG. 23, a robot system 100 in Modification Example 1 has the same basic configuration as the robot system 100 according to Embodiment 6, but Modification Example 1 is different from Embodiment 6 in that a robot 101 is configured by a vertical articulated robot.

The robot system 100 in Modification Example 1, which is configured as described above, also has the similar action and effect to those of the robot system 100 according to Embodiment 6.

### (Embodiment 7)

In a robot system according to Embodiment 7, a robot further includes a first audio input and a first audio output in the robot system according to Embodiment 6 (including the modification example). In addition, a second audio input and a second audio output are further disposed in a second space. A controller is configured to output audio information input to the first audio input, to the second audio output, and output audio information input to the second audio input, to the first audio output.

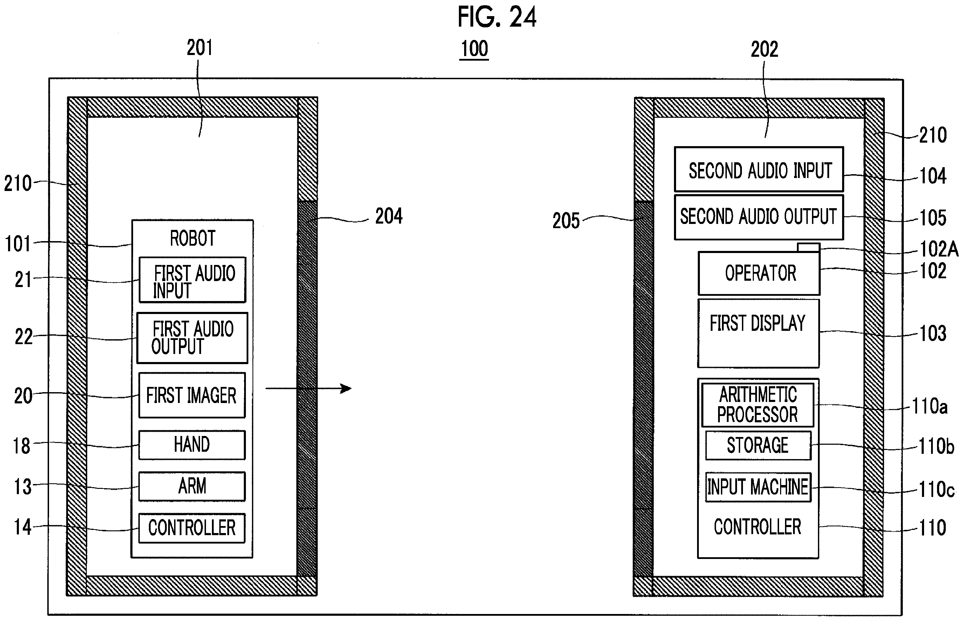

An example of the robot system according to Embodiment 7 will be described below with reference to FIG. 24.

### [Configuration of Robot System]

FIG. 24 is a schematic diagram illustrating a schematic configuration of the robot system according to Embodiment 7.

As illustrated in FIG. 24, the robot system 100 according to Embodiment 7 has the same basic configuration as the robot system 100 according to Embodiment 6. Embodiment 7 is different from Embodiment 6 in that the robot 101 includes a first audio input 21 and a first audio output 22, and that a second audio input 104 and a second audio output 105 are disposed in the second space 202.

The first audio input 21 and the second audio input 104 may be configured, for example, by a microphone. In addition, the first audio output 22 and the second audio output 105 may be configured by a speaker.

The second audio input 104 and the second audio output 105 may be configured by headphones (headsets) with a microphone. In addition, when the first display 103 is configured by a head-mounted display, the second audio input 104 and the second audio output 105 may be configured by a microphone and headphones attached to the head-mounted display.

The robot system 100 in Embodiment 7, which is configured as described above, also has the similar action and effect to those of the robot system 100 according to Embodiment 6.

Further, in the robot system 100 according to Embodiment 7, the robot 101 is provided with the first audio input 21 and the first audio output 22, and the second audio input 104 and the second audio output 105 are disposed in the second space 202. Thus, it is possible to perform communication between the patient and the operator.

As a result, for example, when the operator is a medical staff, or when there is a medical staff beside the operator, it is possible to perform, on the patient, medical practice such as inquiries, auscultation, transmission of test results, communication of treatment policies, and the like.

### (Embodiment 8)

In a robot system according to Embodiment 8, a robot further includes a second display in the robot system according to either Embodiment 6 (including the modification example) or Embodiment 7. In addition, a second imager is further disposed in the second space, and the controller is configured to display image information and/or video information obtained by picking-up of the second imager, on the second display in (α).

In a method for operating a robot system according to Embodiment 8, a robot further includes a second display in the method for operating the robot system according to either Embodiment 6 (including the modification example) or Embodiment 7. In addition, a second imager is further disposed in the second space, and, in (α), the second display is configured to display image information and/or video information obtained by picking-up of the second imager.

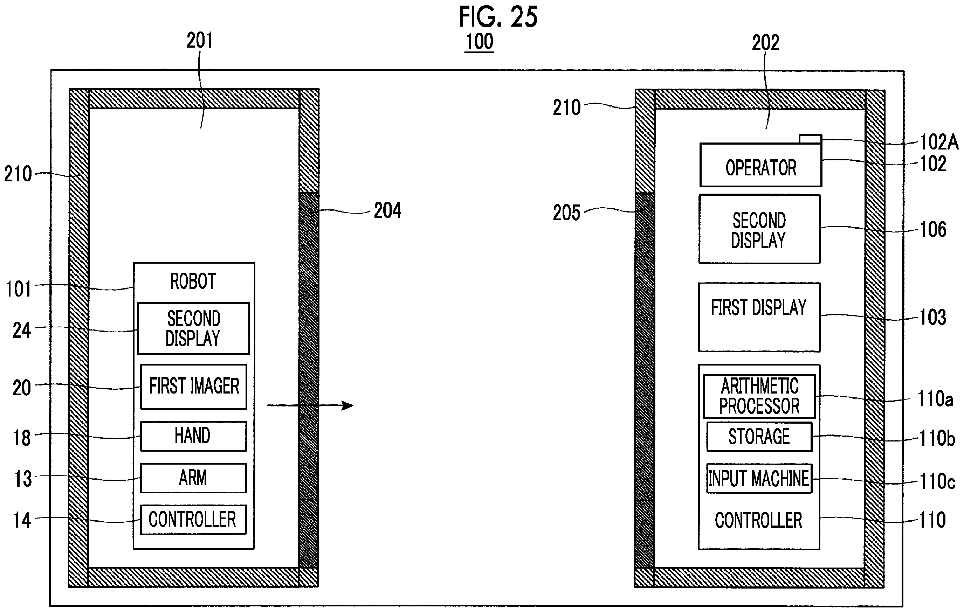

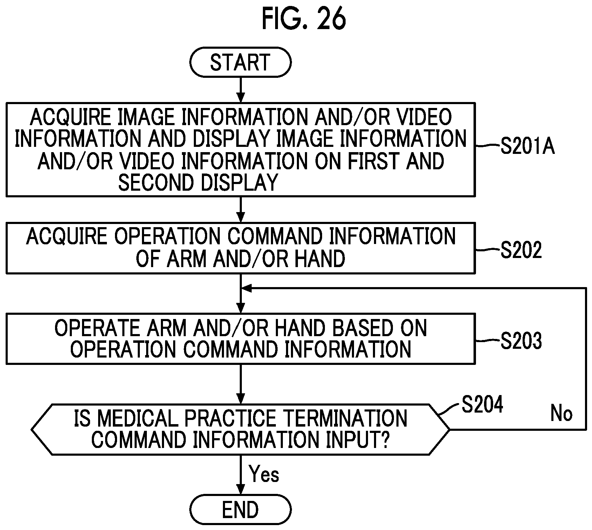

An example of the robot system according to Embodiment 8 will be described below with reference to FIGS. 25 and 26.

### [Configuration of Robot System]

FIG. 25 is a schematic diagram illustrating a schematic configuration of the robot system according to Embodiment 8.

As illustrated in FIG. 25, a robot system 100 according to Embodiment 8 has the same basic configuration as that of the robot system 100 according to Embodiment 6. Embodiment 8 is different from Embodiment 6 in that a robot 101 further includes a second display 24, and that a second imager 106 is further disposed in the second space 202.

The second display 24 is configured to display image information and/or video information obtained by picking-up of the second imager 106. For example, a stationary type display may be configured as the second display 24.

The second imager 106 is configured to pick up an image and/or a video, and output image information and/or video information obtained by pickup to the second display 24 via the controller 110 and the controller 14. For example, a video camera may be used as the second imager 106.

### [Operations, and Actions and Effects of Robot System]

Next, the operation and the action and effect of the robot system 100 according to Embodiment 8 will be described in detail with reference to FIGS. 25 and 26. The following operations are performed by the arithmetic processor 110a of the controller 110 reading the program stored in the storage 110b.

FIG. 26 is a flowchart illustrating an example of an operation of the robot system according to Embodiment 8.

As illustrated in FIG. 26, the operation of the robot system 100 according to Embodiment 8 is basically the same as that of the robot system 100 according to Embodiment 6. Embodiment 8 is different from Embodiment 6 in that the controller 110 executes a process of Step S201A instead of the process of Step S201.

Specifically, the controller 110 acquires the image information and/or the video information obtained by picking-up of the first imager 20, and displays the image information and/or the video information on the first display 103. In addition, the controller 110 acquires the image information and/or the video information obtained by picking-up of the second imager 106, and displays the image information and/or the video information on the second display 24 (Step S201A).

The robot system 100 in Embodiment 8, which is configured as described above, also has the similar action and effect to those of the robot system 100 according to Embodiment 6.

Further, in the robot system 100 according to Embodiment 8, the robot 101 further includes the second display 24, and the second imager 106 is further disposed in the second space 202.

This makes it possible to communicate between the patient and the operator (medical staff).

From the above description, many improvements or other embodiments of the present invention will be apparent to those skilled in the art. Accordingly, the above description should be construed as only an example and is provided for the purpose of teaching, to those skilled in the art, the best forms of carrying out the present invention. Details of the structures and/or the functions can be substantially changed without departing from the present invention.

### Industrial Applicability

According to the robot system and the method for operating the robot system of the present invention, it is possible to sufficiently reduce the infection of a medical staff and the like in a hospital. Thus, the robot system and the method for operating the robot system of the present invention are useful in the field of robots.

### Reference Signs List

2A FIRST MOUNTING PORTION
2B SECOND MOUNTING PORTION
5a FIRST LINK
5b SECOND LINK
12 CARRIAGE
13 ARM
13A FIRST ARM
13B SECOND ARM
14 CONTROLLER
15A FIRST ARM PORTION
15B SECOND ARM PORTION
16 BASE SHAFT
17A FIRST WRIST PORTION
17B SECOND WRIST PORTION
18 HAND
18A FIRST HAND
18B SECOND HAND
19 WHEELS
20 FIRST IMAGER
21 FIRST AUDIO INPUT
22 FIRST AUDIO OUTPUT
23 CONTAINER
24 SECOND DISPLAY
31 MAIN BODY
32 INTERMEDIATE MEMBER
33 HOLDING MEMBER
34 ACTUATOR
35 SUPPORT MEMBER
36 CAMERA
37 CHUCK MECHANISM
38A LASER POINTER
38B LASER POINTER
39B LASER LIGHT
39A LASER LIGHT
50 STERILIZED COTTON SWAB
50A VIRTUAL STERILIZED COTTON SWAB
50B FIRST REGION
60 VIRTUAL PATIENT
100 ROBOT SYSTEM
101 ROBOT
102 OPERATOR
102A RELEASE BUTTON
103 FIRST DISPLAY
103A FIRST VIDEO INFORMATION
103B SECOND VIDEO INFORMATION
103C THIRD VIDEO INFORMATION
103D FOURTH VIDEO INFORMATION
104 SECOND AUDIO INPUT
105 SECOND AUDIO OUTPUT
106 SECOND IMAGE PICKUP
110 CONTROLLER
110a ARITHMETIC PROCESSOR
110b STORAGE
110c INPUT MACHINE
201 FIRST SPACE
202 SECOND SPACE
203 THIRD SPACE
204 SHUTTER
205 SHUTTER
206 SHUTTER
210 PARTITION WALL MEMBER
220 BASE
221 SHIELDING PLATE
222 OPENING
230 POSITIONING DEVICE
231 MAIN BODY
232 ABUTTING TARGET PORTION
233 CHIN REST
300 DISINFECTOR
J1 ROTATING JOINT
J2 ROTATING JOINT
J3 LINEAR MOTION JOINT
J4 ROTATING JOINT
J5 ROTATING JOINT
J6 ROTATING JOINT
L1 ROTATION AXIS
L2 ROTATION AXIS
L3 ROTATION AXIS
L4 ROTATION AXIS
L5 ROTATION AXIS

## Claims

1. A robot system comprising:
a robot including an arm with a hand that holds a medical test instrument and/or a medical examination instrument;
an operator configured to operate the robot; and
a controller, wherein
a first space in which the robot is disposed is isolated from a second space in which the operator is disposed, and
the controller is configured to
(A) cause the robot to be self-propelled to approach the patient, and
(B) operate the arm and/or the hand based on operation command information of the arm and/or the hand, which has been input from the operator, after (A) is performed.

2. The robot system according to claim 1, further comprising:
a first imager; and
a first display configured to display image information and/or video information obtained by picking-up of the first imager, wherein
the controller is configured to perform (B) in a state where the image information and/or video information obtained by picking-up of the first imager is displayed on the first display.

3. The robot system according to claim 1 or 2, wherein
the robot further includes a first audio input and a first audio output,
a second audio input and a second audio output are further disposed in the second space, and
the controller is configured to
output audio information input to the first audio input, to the second audio output, and
output audio information input to the second audio input, to the first audio output.

4. The robot system according to any one of claims 1 to 3, wherein
the robot further includes a container that contains at least a transfer item among a drug, a meal, a test reagent, a specimen, a medical test instrument, and a medical examination instrument.

5. The robot system according to any one of claims 1 to 4, further comprising:
a third space isolated from the first space and the second space, wherein
the robot is disinfected in the third space.

6. The robot system according to claim 5, wherein
the robot is configured to disinfect the robot itself.

7. The robot system according to claim 5, wherein
a disinfector configured to disinfect the robot is disposed in the third space.

8. The robot system according to any one of claims 5 to 7, wherein
the controller is configured to further (C) cause the robot to be self-propelled into the third space and disinfect the robot after (B) is performed.

9. The robot system according to any one of claims 1 to 8, wherein
the robot further includes a second display,
a second imager is further disposed in the second space, and
the controller is configured to display image information and/or video information obtained by picking-up of the second imager, on the second display in (B).

10. The robot system according to any one of claims 1 to 9, wherein
the robot and the operator are configured by a master-slave method.

11. The robot system according to any one of claims 1 to 10, wherein
a pair of laser light indicators are disposed at the hand such that rays of light with which irradiation is performed from the laser light indicators intersect with each other.

12. The robot system according to any one of claims 2 to 11, wherein
the first imager is disposed at the robot.

13. The robot system according to any one of claims 2 to 12, wherein
the first display is configured to display a virtual model showing position information of the medical test instrument and/or the medical examination instrument.

14. The robot system according to any one of claims 2 to 13, wherein
the first display is configured to display a virtual model of a medical practice target site of the patient.

15. The robot system according to any one of claims 1 to 14, wherein
an operation switch configured to perform an instruction to release holding of the medical test instrument and/or the medical examination instrument is disposed at the operator.

16. A robot system comprising:
a robot including an arm with a hand that holds a medical test instrument and/or a medical examination instrument;
an operator configured to operate the robot; and
a controller, wherein
a first space in which the robot is disposed is isolated from a second space in which the operator is disposed,
a first imager is disposed at the hand, and
the controller is configured to (α) operate the arm and/or the hand based on operation command information of the arm and/or the hand, which has been input from the operator.

17. The robot system according to claim 16, further comprising:
a first display configured to display image information and/or video information obtained by picking-up of the first imager, wherein
the controller is configured to perform (α) in a state where the image information and/or the video information obtained by picking-up of the first imager is displayed on the first display.

18. The robot system according to claim 16 or 17, wherein
a pair of laser light indicators are disposed at the hand such that rays of light with which irradiation is performed from the laser light indicators intersect with each other.

19. The robot system according to any one of claims 16 to 18, wherein
the robot further includes a first audio input and a first audio output,
a second audio input and a second audio output are further disposed in the second space, and
the controller is configured to
output audio information input to the first audio input, to the second audio output, and
output audio information input to the second audio input, to the first audio output.

20. The robot system according to any one of claims 16 to 19, wherein
the robot further includes a second display,
a second imager is further disposed in the second space, and
the controller is configured to display image information and/or video information obtained by picking-up of the second imager, on the second display in (α).

21. The robot system according to any one of claims 16 to 20, wherein
the robot and the operator are configured by a master-slave method.

22. The robot system according to any one of claims 17 to 21, wherein
the first display is configured to display a virtual model showing position information of the medical test instrument and/or the medical examination instrument.

23. The robot system according to any one of claims 17 to 22, wherein
the first display is configured to display a virtual model of a medical practice target site of a patient.

24. The robot system according to any one of claims 16 to 23, wherein
an operation switch configured to perform an instruction to release holding of the medical test instrument and/or the medical examination instrument is disposed at the operator.

25. A method for operating a robot system that includes a robot including an arm with a hand that holds a medical test instrument and/or a medical examination instrument, and an operator configured to operate the robot, wherein
a first space in which the robot is disposed is isolated from a second space in which the operator is disposed, and
the method comprises:
(A) automatically moving the robot to a vicinity of a patient based on position information of the patient, which has been input from the operator; and
(B) operating the arm and/or the hand based on operation command information of the arm and/or the hand, which has been input from the operator, after (A) is performed.

26. The method for operating a robot system according to claim 25, wherein
the robot system further includes
a first imager, and
a first display configured to display image information and/or video information obtained by picking-up of the first imager, and
(B) is performed in a state where the image information and/or the video information obtained by picking-up of the first imager is displayed on the first display.

27. The method for operating a robot system according to claim 25 or 26, wherein
the robot further includes a first audio input and a first audio output,
a second audio input and a second audio output are further disposed in the second space,
the first audio output is configured to output audio information input to the second audio input, and
the second audio output is configured to output audio information input to the first audio input.

28. The method for operating a robot system according to any one of claims 25 to 27, wherein
the robot further includes a container that contains at least a transfer item among a drug, a meal, a test reagent, a specimen, a medical test instrument, and a medical examination instrument.

29. The method for operating a robot system according to any one of claims 25 to 28, wherein
a third space isolated from the first space and the second space is further provided, and
the robot is disinfected in the third space.

30. The method for operating a robot system according to claim 29, wherein
the robot is configured to disinfect the robot itself.

31. The method for operating a robot system according to claim 29, wherein
a disinfector configured to disinfect the robot is disposed in the third space.

32. The method for operating a robot system according to any one of claims 29 to 31, further comprising:
(C) causing the robot to be self-propelled into the third space and disinfecting the robot, after (B).

33. The method for operating a robot system according to any one of claims 25 to 32, wherein
the robot further includes a second display,
a second imager is further disposed in the second space, and
the second display is configured to display image information and/or video information obtained by picking-up of the second imager, in (B).

34. The method for operating a robot system according to any one of claims 25 to 33, wherein
the robot and the operator are configured by a master-slave method.

35. The method for operating a robot system according to any one of claims 25 to 34, wherein
a pair of laser light indicators are disposed at the hand such that rays of light with which irradiation is performed from the laser light indicators intersect with each other.

36. The method for operating a robot system according to any one of claims 26 to 35, wherein
the first imager is disposed at the robot.

37. The method for operating a robot system according to any one of claims 26 to 36, wherein
the first display is configured to display a virtual model showing position information of the medical test instrument and/or the medical examination instrument.

38. The method for operating a robot system according to any one of claims 26 to 37, wherein
the first display is configured to display a virtual model of a medical practice target site of the patient.

39. The method for operating a robot system according to any one of claims 25 to 38, wherein
an operation switch configured to perform an instruction to release holding of the medical test instrument and/or the medical examination instrument is disposed at the operator.

40. A method for operating a robot system that includes a robot including an arm with a hand that holds a medical test instrument and/or a medical examination instrument, and an operator configured to operate the robot, wherein
a first space in which the robot is disposed is isolated from a second space in which the operator is disposed,
a first imager is disposed at the hand, and
the method comprises:
(α) operating the arm and/or the hand based on operation command information of the arm and/or the hand, which has been input from the operator.

41. The method for operating a robot system according to claim 40, wherein
the robot system further includes a first display configured to display image information and/or video information obtained by picking-up of the first imager, and
(α) is performed in a state where the image information and/or the video information obtained by picking-up of the first imager is displayed on the first display.

42. The method for operating a robot system according to claim 40 or 41, wherein
a pair of laser light indicators are disposed at the hand such that rays of light with which irradiation is performed from the laser light indicators intersect with each other.

43. The method for operating a robot system according to any one of claims 40 to 42, wherein
the robot further includes a first audio input and a first audio output,
a second audio input and a second audio output are further disposed in the second space,
the first audio output is configured to output audio information input to the second audio input, and
the second audio output is configured to output audio information input to the first audio input.

44. The method for operating a robot system according to any one of claims 40 to 43, wherein
the robot further includes a second display,
a second imager is further disposed in the second space, and
the second display is configured to display image information and/or video information obtained by picking-up of the second imager, in (α).

45. The method for operating a robot system according to any one of claims 40 to 44, wherein
the robot and the operator are configured by a master-slave method.

46. The method for operating a robot system according to any one of claims 41 to 45, wherein
the first display is configured to display a virtual model showing position information of the medical test instrument and/or the medical examination instrument.

47. The method for operating a robot system according to any one of claims 41 to 46, wherein
the first display is configured to display a virtual model of a medical practice target site of a patient.

48. The method for operating a robot system according to any one of claims 40 to 47, wherein
an operation switch configured to perform an instruction to release holding of the medical test instrument and/or the medical examination instrument is disposed at the operator.
